# EUROPÄISCHE PATENTSCHRIFT

(11) **EP 2 220 034 B1**
(45) Veröffentlichungstag und Bekanntmachung des Hinweises auf die Patenterteilung: **23.12.2015**
(21) Anmeldenummer: 08849259.0
(22) Anmeldetag: 13.11.2008
(51) Int. Cl.: C07C 251/08, C08G 18/32, C09J 175/02

(54) **AROMATISCHE ALDIMINE UND ALDIMIN ENTHALTENDE POLYURETHANZUSAMMENSETZUNGEN**
AROMATIC ALDIMINES AND POLYURETHANE COMPOSITIONS WHICH CONTAIN ALDIMINE
ALDIMINES AROMATIQUES ET COMPOSITIONS DE POLYURÉTHANE CONTENANT DE L'ALDIMINE

(30) Priorität: 13.11.2007 EP 07120595
(43) Veröffentlichungstag der Anmeldung: 25.08.2010
(73) Patentinhaber: Sika Technology AG, 6340 Baar (CH)
(72) Erfinder: BURCKHARDT, Urs, CH-8049 Zürich (CH)
(74) Vertreter: Sika Patent Attorneys
(86) Internationale Anmeldenummer: PCT/EP2008/065431
(87) Internationale Veröffentlichungsnummer: WO 2009/062985

(56) Entgegenhaltungen:
- WO-A-2004/013088
- DE-A1- 3 133 769
- US-A- 4 720 535

## Beschreibung

### Technisches Gebiet

Die vorliegende Erfindung betrifft das Gebiet der Aldimine und der feuchtigkeitshärtenden Polyurethanzusammensetzungen, sowie deren Verwendung, insbesondere als elastische Klebstoffe, Dichtstoffe und Beschichtungen.

### Stand der Technik

Aldimine sind Kondensationsprodukte aus primären Aminen und Aldehyden und stellen eine seit langem bekannte Stoffklasse dar. Bei Kontakt mit Wasser können Aldimine zu Aminen und Aldehyden hydrolysieren. Aufgrund dieser Eigenart können sie als geschützte Form von Aminen, beziehungsweise von Aldehyden, verwendet werden. So werden Aldimine beispielsweise in der Polyurethanchemie eingesetzt, wo sie als durch Feuchtigkeit aktivierbare Vernetzer, sogenannte "blockierte Amine" oder "latente Härter", für ein- oder zweikomponentige Isocyanatgruppen aufweisende Zusammensetzungen dienen.

Isocyanatgruppen aufweisende Zusammensetzungen, auch als Polyurethanzusammensetzungen bezeichnet, werden für verschiedenste Verwendungszwecke eingesetzt, unter anderem als ein- und zweikomponentige elastische Klebstoffe, Dichtstoffe oder Beschichtungen. Sie weisen eine besonders gute mechanische Festigkeit und thermische Beständigkeit auf, wenn zu ihrer Aushärtung zumindest teilweise aromatische Polyamine, insbesondere solche mit primären Aminogruppen, eingesetzt werden. Insbesondere in bei Raumtemperatur verwendeten Polyurethanzusammensetzungen wird der Einsatz von primären aromatischen Polyaminen als Härter allerdings durch die Tatsache erschwert, dass diese meist relativ hochschmelzende und oft schwerlösliche Feststoffe darstellen. Dies gilt insbesondere für sterisch ungehinderte aromatische Polyamine wie beispielsweise 1,4-Phenylendiamin, das einen Schmelzpunkt von über 140 °C und eine geringe Löslichkeit besitzt und damit in freier Form kaum einsetzbar ist. Gerade dieses Polyamin wäre aber aufgrund seines niedrigen Amin-Equivalenzgewichts, der sterisch ungehinderten und in para-Stellung zueinander stehenden Aminogruppen, die ihm eine hohe Reaktivität verleihen und zu hoher mechanischen Festigkeit führen, sowie seiner für aromatische Polyamine vergleichsweise niedrigen Toxizität als Härter für Polyurethanzusammensetzungen besonders attraktiv. Bei Raumtemperatur flüssige primäre aromatische Polyamine wie 3,5-Diethyl-2,4-diaminotoluol oder 3,5-Dimethylthio-2,4-diaminotoluol weisen im allgemeinen sterisch gehinderte, relativ langsam reagierende Aminogruppen auf und führen zu einer deutlich geringeren mechanischen Festigkeit.

Blockierte primäre aromatische Polyamine in Form von Aldiminen und ihre Verwendung als Härter für Polyurethanzusammensetzungen sind beispielsweise aus US 4,720,535 und DE 31 33 769 A1 bekannt. Die dort beschriebenen aromatischen Aldimine weisen den Nachteil auf, dass sie bei ihrer Hydrolyse und damit bei der Aushärtungsreaktion flüchtige, geruchsintensive Aldehyde freisetzen. Zudem stellen sie wie die freien Polyamine zumeist Feststoffe mit relativ hohem Schmelzpunkt dar oder sind zumindest hochviskos und sind deshalb bei Raumtemperatur nur erschwert einsetzbar.

### Darstellung der Erfindung

Aufgabe der vorliegenden Erfindung ist es daher, aromatische Aldimine zur Verfügung zu stellen, welche auf bei Raumtemperatur festen primären aromatischen Polyaminen beruhen, bei Raumtemperatur flüssig und möglichst niedrigviskos sind, geruchsarm hydrolysieren und als blockierte Amine in härtbaren Zusammensetzungen, insbesondere in ein- und zweikomponentigen Isocyanatgruppen aufweisenden Zusammensetzungen, eingesetzt werden können.

Überraschenderweise wurde gefunden, dass aromatische Dialdimine nach Anspruch 1 diese Aufgabe lösen. Durch die Umsetzung von ausgewählten, bei Raumtemperatur festen primären aromatischen Diaminen mit speziellen, Estergruppen-haltigen Aldehyden sind aromatische Dialdimine erhältlich, welche geruchsarm oder geruchsfrei hydrolysieren und überraschenderweise bei Raumtemperatur flüssig und dabei sogar meist niedrigviskos sind. Dadurch können sie auf einfache Weise als Bestandteil von härtbaren Zusammensetzungen, welche bei Raumtemperatur hergestellt und / oder verwendet werden, eingesetzt werden. Zusammen mit Reaktivgruppen, insbesondere zusammen mit Isocyanatgruppen, weisen sie eine gute Lagerstabilität auf.

Ein weiterer Gegenstand der Erfindung sind härtbare Zusammensetzungen gemäss Anspruch 4 enthaltend die beschriebenen aromatischen Dialdimine. Diese Zusammensetzungen weisen insbesondere mindestens ein Polyisocyanat P auf und weisen eine relativ lange Offenzeit auf, härten aber überraschenderweise trotzdem schnell aus, wobei dies ohne starke Geruchsbildung und ohne Blasenbildung vonstatten geht. Zudem verfügen sie nach der Aushärtung über ausgezeichnete mechanische Eigenschaften, insbesondere eine hohe Festigkeit bei einer hohen Dehnbarkeit, und eine gute Beständigkeit gegenüber Wärme und Feuchtigkeit.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung sind aromatische Dialdimine der Formel (I).

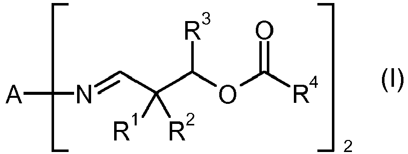

Hierbei steht A für den Rest eines primären aromatischen Diamins B nach der Entfernung der zwei primären Aminogruppen, wobei das Diamin B ausgewählt ist aus der Gruppe bestehend aus 1,2-, 1,3- und 1,4-Phenylendiamin, 2,4- und 2,6-Toluylendiamin (TDA), 3,4-Toluylendiamin, 5-Isopropyl-2,4-toluylendiamin, 5-(tert.Butyl)-2,4-toluylendiamin, 4,6-Dialkyl-1,3-phenylendiamine mit Alkylgruppen, die insbesondere Methyl-, Ethyl-, Isopropyl- oder 1-Methylpropyl-Gruppen sind, 4,4'-, 2,4'- und 2,2'-Diaminodiphenylmethan, 3,3'-Dimethyl-4,4'-diaminodiphenylmethan, 3,3'-Dichlor-4,4'-diaminodiphenylmethan (MOCA), 3,3'-Di-tert.butyl-4,4'-diaminodiphenylmethan, 5,5'-Methylendianthranilsäure, Dimethyl-(5,5'-methylendianthranilat), 1,3-Propylen-bis-(4-aminobenzoat), 1,4-Butylen-bis-(4-aminobenzoat) und 1,2-Bis-(2-aminophenylthio)-ethan.

Weiterhin stehen R¹ und R² entweder unabhängig voneinander jeweils für einen einwertigen Kohlenwasserstoffrest mit 1 bis 12 C-Atomen, oder R¹ und R² stehen zusammen für einen zweiwertigen Kohlenwasserstoffrest mit 4 bis 20 C-Atomen, der Teil eines, gegebenenfalls substituierten, carbocyclischen Rings mit 5 bis 8, bevorzugt 6, C-Atomen ist.

Weiterhin steht R³ für ein Wasserstoffatom oder für eine Alkylgruppe oder für eine Cycloalkylgruppe oder für eine Arylalkylgruppe, insbesondere mit 1 bis 12 C-Atomen.

Weiterhin steht R⁴ entweder für einen linearen oder verzweigten Alkylrest mit 6 bis 20 C-Atomen, gegebenenfalls mit cyclischen Anteilen und gegebenenfalls mit mindestens einem Heteroatom, insbesondere in der Form von Ether-, Ester- oder Aldehyd-Sauerstoff,
oder R⁴ steht für einen einfach oder mehrfach ungesättigten, linearen oder verzweigten Kohlenwasserstoffrest mit 6 bis 20 C-Atomen.

Die Dialdimine der Formel (I) sind bei Raumtemperatur flüssig.

Der Begriff "primäre Aminogruppe" bezeichnet im vorliegenden Dokument eine NH₂-Gruppe, die an einen organischen Rest gebunden ist, während der Begriff "sekundäre Aminogruppe" eine NH-Gruppe bezeichnet, die an zwei organische Reste, welche auch gemeinsam Teil eines Rings sein können, gebunden ist. Als "primäres Diamin" wird ein Amin mit zwei primären Aminogruppen bezeichnet.

Als "aromatische Aminogruppe" wird eine Aminogruppe bezeichnet, die an einen aromatischen oder heteroaromatischen Rest gebunden ist. Als "aromatisches Amin" wird eine organische Verbindung bezeichnet, welche ausschliesslich aromatische Aminogruppen aufweist.

Als "Raumtemperatur" wird eine Temperatur von 23 °C bezeichnet.

Bevorzugt stehen R¹ und R² in Formel (I) jeweils für eine Methylgruppe.

Weiterhin bevorzugt steht R³ in Formel (I) für ein Wasserstoffatom.

Weiterhin bevorzugt steht R⁴ in Formel (I)
entweder für einen linearen oder verzweigten Alkylrest mit 11 bis 20 C-Atomen, gegebenenfalls mit cyclischen Anteilen und gegebenenfalls mit mindestens einem Heteroatom, insbesondere in der Form von Ether-, Ester- oder Aldehyd-Sauerstoff,
oder für einen einfach oder mehrfach ungesättigten, linearen oder verzweigten Kohlenwasserstoffrest mit 11 bis 20 C-Atomen.

Besonders bevorzugt steht R⁴ in Formel (I) für einen Alkylrest mit 11 C-Atomen.

Weiterhin bevorzugt steht A in Formel (I) für den Rest eines primären aromatischen Diamins **B** nach der Entfernung der zwei primären Aminogruppen, wobei das Diamin **B** ausgewählt ist aus der Gruppe bestehend aus 1,2-, 1,3- und 1,4-Phenylendiamin, 2,4- und 2,6-Toluylendiamin, 3,4-Toluylendiamin, 4,4'-, 2,4'- und 2,2'-Diaminodiphenylmethan und 3,3'-Dichlor-4,4'-diaminodiphenylmethan.

Als Diamin **B** besonders bevorzugt sind 1,3-Phenylendiamin, 1,4-Phenylendiamin, 2,4-Toluylendiamin und 4,4'-Diaminodiphenylmethan.

Als Diamin **B** am meisten bevorzugt ist 1,4-Phenylendiamin.

Bevorzugte Dialdimine der Formel (I) sind 1,3- und 1,4-Bis-((2,2-dimethyl-3-lauroyloxy-propyliden)amino)-benzol, 2,4-Bis-((2,2-dimethyl-3-lauroyloxy-propyliden)amino)-toluol und 4,4'-Bis-((2,2-dimethyl-3-lauroyloxy-propyliden)amino)-diphenylmethan.

Ein besonders bevorzugtes Dialdimin der Formel (I) ist 1,4-Bis-((2,2-dimethyl-3-lauroyloxy-propyliden)amino)-benzol.

Ein aromatisches Dialdimin der Formel (I) ist erhältlich durch eine Kondensationsreaktion unter Abspaltung von Wasser zwischen mindestens einem bei Raumtemperatur festen, primären aromatischen Diamin **B** der Formel (II) und mindestens einem bei Raumtemperatur flüssigen Aldehyd **ALD** der Formel (III). Der Aldehyd **ALD** der Formel (III) wird hierbei in Bezug auf die Aminogruppen des Diamins stöchiometrisch oder in stöchiometrischem Überschuss eingesetzt. In einem bevorzugten Verfahren wird das Diamin **B** ohne die Verwendung von Lösemitteln mit dem Aldehyd **ALD** der Formel (III) umgesetzt und das entstehende Wasser mittels Vakuum aus der Reaktionsmischung entfernt.

A⁅NH₂]₂ (II)

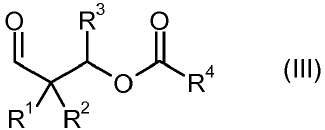

In den Formeln (II) und (III) weisen A, R¹, R², R³ und R⁴ die bereits erwähnten Bedeutungen auf.

Der zur Herstellung eines aromatischen Dialdimins der Formel (I) einsetzbare Aldehyd **ALD** der Formel (III) ist bei Raumtemperatur flüssig und stellt einen tertiären aliphatischen oder tertiären cycloaliphatischen Aldehyd dar. Der Aldehyd **ALD** der Formel (III) ist ein Ester, welcher insbesondere erhältlich ist aus der Reaktion von 2,2-disubstituierten 3-Hydroxyaldehyden mit geeigneten Carbonsäuren.

Für diese Umsetzung geeignete Carbonsäuren sind beispielsweise gesättigte aliphatische Carbonsäuren, wie 2-Ethylcapronsäure, Oenanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure; einfach ungesättigte aliphatische Carbonsäuren wie Palmitoleinsäure, Ölsäure; mehrfach ungesättigte aliphatische Carbonsäuren wie Linolsäure, Linolensäure, Elaeostearinsäure, Arachidonsäure; cycloaliphatische Carbonsäuren wie Cyclohexancarbonsäure, Fettsäuregemische aus der technischen Verseifung von natürlichen Ölen und Fetten wie beispielsweise Rapsöl, Sonnenblumenöl, Leinöl, Ölbaumöl, Kokosnussöl, Ölpalmkernöl und Ölpalmöl; sowie Dicarbonsäuremonoalkyl- und -aryl-ester, wie sie aus der einfachen Veresterung von Dicarbonsäuren wie Bernsteinsäure, Glutarsäure, Adipinsäure, Pimelinsäure, Korksäure, Azelainsäure, Sebacinsäure, 1,12-Dodecandisäure, Maleinsäure, Fumarsäure, Hexahydrophthalsäure, Hexahydroisophthalsäure, Hexahydroterephthalsäure, 3,6,9-Trioxaundecandisäure und ähnliche Derivate von Polyethylenglykol, mit Alkoholen wie Methanol, Ethanol, Propanol, Butanol, höheren Homologen und Isomeren dieser Alkohole erhalten werden.

Für diese Umsetzung geeignete 2,2-disubstituierte 3-Hydroxyaldehyde sind beispielsweise 2,2-Dimethyl-3-hydroxypropanal, 2-Hydroxymethyl-2-methyl-butanal, 2-Hydroxymethyl-2-ethyl-butanal, 2-Hydroxymethyl-2-methylpentanal, 2-Hydroxymethyl-2-ethyl-hexanal, 1-Hydroxymethyl-cyclopentancarboxaldehyd, 1-Hydroxymethyl-cyclohexancarboxaldehyd 1-Hydroxymethylcyclohex-3-encarboxaldehyd, 2-Hydroxymethyl-2-methyl-3-phenyl-propanal, 3-Hydroxy-2-methyl-2-phenyl-propanal und 3-Hydroxy-2,2-diphenyl-propanal.

Solche 2,2-disubstituierten 3-Hydroxyaldehyde sind ihrerseits erhältlich aus Aldol-Reaktionen, insbesondere gekreuzten Aldol-Reaktionen, zwischen primären oder sekundären aliphatischen Aldehyden, insbesondere Formaldehyd, und sekundären aliphatischen, sekundären arylaliphatischen oder sekundären cycloaliphatischen Aldehyden, wie beispielsweise Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd (Hydratropaldehyd) oder Diphenylacetaldehyd.

Bevorzugte Aldehyde **ALD** der Formel (III) sind 3-Cyclohexanoyloxy-2,2-dimethylpropanal, 2,2-Dimethyl-3-(2-ethylhexyloxy)-propanal, 2,2-Dimethyl3-lauroyloxy-propanal, 2,2-Dimethyl-3-myristoyloxypropanal, 2,2-Dimethyl-3-palmitoyloxy-propanal, 2,2-Dimethyl-3-stearoyloxy-propanal sowie analoge Ester anderer 2,2-disubstituierter 3-Hydroxyaldehyde.

Besonders bevorzugt als Aldehyd **ALD** der Formel (III) ist 2,2-Dimethyl-3-lauroyloxypropanal.

In einer bevorzugten Herstellmethode eines Aldehyds **ALD** der Formel (III) wird ein 2,2-disubstituierter 3-Hydroxyaldehyd, beispielsweise 2,2-Dimethyl-3-hydroxypropanal, welcher beispielsweise aus Formaldehyd (oder Paraformaldehyd) und Isobutyraldehyd, gegebenenfalls in situ, hergestellt werden kann, mit einer Carbonsäure zum entsprechenden Ester umgesetzt. Diese Veresterung kann ohne die Verwendung von Lösemitteln nach bekannten Methoden erfolgen. Diese sind beispielsweise beschrieben in Houben-Weyl, "Methoden der organischen Chemie", Vol. VIII, Seiten 516 - 528.

Der Aldehyd **ALD** der Formel (III) ist geruchsarm oder geruchsfrei.

In einer bevorzugten Ausführungsform ist der Aldehyde **ALD** der Formel (III) geruchsfrei. Geruchsfreie Aldehyde **ALD** der Formel (III) stellen Aldehyde **ALD1** der Formel (III a) dar, 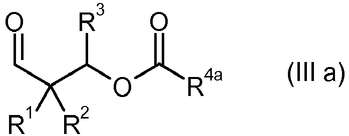 wobei R^{4a}
entweder für einen linearen oder verzweigten Alkylrest mit 11 bis 20 C-Atomen, gegebenenfalls mit cyclischen Anteilen und gegebenenfalls mit mindestens einem Heteroatom, insbesondere Sauerstoff in Form von Ether-, Ester- oder Aldehydgruppen,
oder für einen einfach oder mehrfach ungesättigten, linearen oder verzweigten Kohlenwasserstoffrest mit 11 bis 20 C-Atomen steht,
und R¹, R² und R³ die bereits genannten Bedeutungen aufweisen.

Diese bevorzugten Aldehyde **ALD1** der Formel (III a) sind geruchsfrei und sind bei Raumtemperatur flüssig.

Beispiele für solche geruchsfreien Aldehyde **ALD1** sind Veresterungsprodukte aus den bereits genannten 2,2-disubstituierten 3-Hydroxyaldehyden mit Carbonsäuren wie beispielsweise Laurinsäure, Tridecansäure, Myristinsäure, Pentadecansäure, Palmitinsäure, Margarinsäure, Stearinsäure, Nonadecansäure, Arachinsäure, Palmitoleinsäure, Ölsäure, Linolsäure, Linolensäure, Elaeostearinsäure, Arachidonsäure, Fettsäuren aus der technischen Verseifung von natürlichen Ölen und Fetten, wie beispielsweise Rapsöl, Sonnenblumenöl, Leinöl, Ölbaumöl, Kokosnussöl, Ölpalmkernöl und Ölpalmöl, sowie technische Gemische von Fettsäuren, welche diese Säuren enthalten. Bevorzugte geruchsfreie Aldehyde der Formel (III a) sind 2,2-Dimethyl-3-lauroyloxypropanal, 2,2-Dimethyl-3-myristoyloxypropanal, 2,2-Dimethyl-3-palmitoyloxypropanal und 2,2-Dimethyl-3-stearoyloxypropanal. Besonders bevorzugt ist 2,2-Dimethyl-3-lauroyloxypropanal.

Die Dialdimine der Formel (I) haben die Eigenschaft, dass sie in Abwesenheit von Wasser nicht in nennenswertem Ausmass mit Isocyanaten reagieren. Ihre Reste A, R¹, R², R³ und R⁴ weisen keine Gruppierungen auf, welche mit Isocyanatgruppen in nennenswertem Ausmass reagieren. Insbesondere weisen A, R¹, R², R³ und R⁴ keine Hydroxylgruppen, keine primären oder sekundären Aminogruppen und keine Mercaptogruppen auf.

Die Dialdimine der Formel (I) enthalten sterisch gehinderte Aldiminogruppen, welche am in α-Stellung stehenden C-Atom kein Wasserstoffatom aufweisen und deshalb nicht zu Enaminogruppen tautomerisieren können. Aufgrund dieser Eigenschaft bilden sie zusammen mit Isocyanatgruppen besonders lagerfähige, das heisst weitgehend viskositätsstabile, Mischungen.

Bei den Dialdiminen der Formel (I) handelt es sich überraschenderweise um bei Raumtemperatur flüssige Substanzen, obwohl sie abgeleitet sind von den genannten Diaminen **B,** welche bei Raumtemperatur fest sind. Für viele Anwendungen ist ein bei Raumtemperatur flüssiger Aggregatszustand ein grosser Vorteil, da solche Substanzen ohne vorheriges Lösen oder Aufschmelzen in Zusammensetzungen einsetzbar sind, insbesondere für Anwendungen in Zusammensetzungen, welche bei Raumtemperatur oder nur leicht erhöhten Temperaturen hergestellt und/oder appliziert werden, und welche ihrerseits einen flüssigen oder pastösen Aggregatszustand aufweisen. Insbesondere überraschend ist die Tatsache, dass es sich auch bei Dialdiminen der Formel (I), welche abgeleitet sind von Diaminen **B** mit einer schlechten Löslichkeit und mit einem hohen Schmelzpunkt, wie beispielsweise 1,4-Phenylendiamin mit einem Schmelzpunkt von über 140 °C, um bei Raumtemperatur flüssige Verbindungen handelt. Bevorzugte Dialdimine der Formel (I) sind niedrigviskose Flüssigkeiten und weisen bei 20 °C eine Viskosität von höchstens 1000 mPa·s, insbesondere eine Viskosität zwischen 5 und 500 mPa·s, auf. Mit dem Begriff "Viskosität" wird im vorliegenden Dokument die dynamische Viskosität oder Scherviskosität bezeichnet, welche durch das Verhältnis zwischen der Schubspannung und der Scherrate (Geschwindigkeitsgefälle) definiert ist und wie in DIN EN ISO 3219 beschrieben bestimmt wird. Eine niedrige Viskosität ist für viele Anwendungen der Dialdimine der Formel (I) ein zusätzlicher Vorteil, insbesondere für Anwendungen in Zusammensetzungen, welche ihrerseits eine nicht zu hohe Viskosität aufweisen sollen.

Bei den Dialdiminen der Formel (I) handelt es sich weiterhin um geruchsarme oder geruchsfreie Substanzen. Bevorzugt sind geruchsfreie Dialdimine der Formel (I), welche ausgehend von den bevorzugten geruchsfreien Aldehyden **ALD1** der Formel (III a) hergestellt sind. Für viele Anwendungen ist die Geruchsfreiheit ein grosser Vorteil oder eine unabdingbare Voraussetzung, insbesondere in geschlossenen Räumen wie im Innern von Gebäuden oder Fahrzeugen und bei grossflächigen Applikationen wie beispielsweise beim Aufbringen von Bodenbelägen.

Die Dialdimine der Formel (I) sind unter geeigneten Bedingungen lagerstabil. Bei Zutritt von Feuchtigkeit können ihre Aldiminogruppen über Zwischenstufen formal zu Aminogruppen hydrolysieren, wobei die entsprechenden, zur Herstellung der Dialdimine verwendeten, Aldehyde **ALD** der Formel (III) freigesetzt werden, welche, wie bereits beschrieben, geruchsarm oder geruchsfrei sind. Da diese Hydrolysereaktion reversibel ist und das chemische Gleichgewicht deutlich auf der Aldiminseite liegt, ist davon auszugehen, dass in Abwesenheit von gegenüber Aminen reaktiven Verbindungen nur ein Teil der Aldiminogruppen teilweise oder ganz hydrolysieren.

Die Dialdimine der Formel (I) sind aus gut erhältlichen AusgangsSubstanzen in einem relativ einfachen Verfahren herstellbar. Obwohl die bei ihrer Herstellung verwendeten Diamine **B** bei Raumtemperatur fest sind, sind die entsprechenden Dialdimine der Formel (I), wie bereits erwähnt, bei Raumtemperatur flüssige Verbindungen.

Die Dialdimine der Formel (I) lassen sich sehr breit verwenden. Sie lassen sich zum Beispiel überall dort einsetzen, wo sie als Quelle von Aldehyden **ALD** der Formel (III) oder von Diaminen **B** der Formel (II) dienen können. Insbesondere können sie in der Funktion von geschützten Aminen, beziehungsweise geschützten Aldehyden, in Aldehyd- und/oder Amin-reaktiven Systemen eingesetzt werden und dort bei Bedarf gezielt entschützt werden. Insbesondere finden sie in Systemen Anwendung, in welchen Verbindungen vorhanden sind, welche mit primären Aminen reagieren. Die Entschützung erfolgt hydrolytisch, beispielsweise durch Kontakt mit Wasser oder Feuchtigkeit, insbesondere Luftfeuchtigkeit.

Besonders geeignet sind die Dialdimine der Formel (I) als Bestandteil von Zusammensetzungen basierend auf mindestens einem Isocyanat und/oder mindestens einem Epoxidharz, insbesondere für Anwendungen wie Klebstoffe, Dichtstoffe, Vergussmassen, Beschichtungen, Bodenbeläge, Anstriche, Lacke, Primer oder Schäume, insbesondere, wenn diese Zusammensetzungen bei Raumtemperatur eine tiefe Viskosität aufweisen sollen. Durch den Einsatz der bei Raumtemperatur flüssigen Dialdimine der Formel (I) sind Zusammensetzungen zugänglich, die formal über primäre aromatische Diamine, insbesondere sterisch wenig oder nicht gehinderte primäre aromatische Diamine, welche als solche relativ hochschmelzende und oft schwerlösliche Feststoffe darstellen, aushärten. Durch den Einsatz solcher Diamine in Form von daraus erhältlichen Dialdiminen der Formel (I) stellt der hohe Schmelzpunkt und/oder die schlechte Löslichkeit der Diamine selbst auch bei Raumtemperatur keine Schwierigkeit mehr dar. In Form seines Dialdimins der Formel (I) lässt sich insbesondere auch das besonders hochschmelzende und schwerlösliche 1,4-Phenylendiamin auf einfache Weise einsetzen.

Insbesondere eignen sich die Dialdimine der Formel (I) als Härter für ein- oder zweikomponentige, Isocyanatgruppen aufweisende Zusammensetzungen wie Klebstoffe, Dichtstoffe, Vergussmassen, Beschichtungen, Bodenbeläge, Anstriche, Lacke, Primer oder Schäume.

Zusammensetzungen aus Isocyanatgruppen aufweisenden Verbindungen und Dialdiminen der Formel (I) reagieren bei Kontakt mit Wasser bzw. Feuchtigkeit unter Hydrolyse der Aldiminogruppen zu Harnstoffgruppen aufweisenden Verbindungen. Die Isocyanatgruppen reagieren dabei mit den durch die Hydrolyse der Aldiminogruppen formal frei werdenden primären Aminogruppen, wobei ein Aldehyd **ALD** freigesetzt wird. Im Verhältnis zu den Aldiminogruppen überschüssige Isocyanatgruppen reagieren direkt mit Feuchtigkeit und bilden ebenfalls Harnstoffgruppen. Bei geeigneter Stöchiometrie zwischen Isocyanatgruppen und Aldiminogruppen härtet die Zusammensetzung als Ergebnis dieser Reaktionen aus; dieser Prozess wird auch als Vernetzung bezeichnet. Die Reaktion der Isocyanatgruppen mit den hydrolysierenden Aldiminogruppen muss dabei nicht notwendigerweise über freie Aminogruppen erfolgen. Selbstverständlich sind auch Reaktionen mit Zwischenstufen der Hydrolysereaktion möglich. Beispielsweise ist es denkbar, dass eine hydrolysierende Aldiminogruppe in der Form eines Halbaminals direkt mit einer Isocyanatgruppe reagiert.

Dialdimine der Formel (I), welche ausgehend 1,4-Phenylendiamin, dem am meisten bevorzugten Diamin **B,** hergestellt sind, sind ganz besonders geeignet als Bestandteil von Isocyanatgruppen aufweisenden Zusammensetzungen. Von 1,4-Phenylendiamin abgeleitete Dialdimine der Formel (I) stellen bei Raumtemperatur überraschenderweise niedrigviskose Flüssigkeit mit einer gegenüber Isocyanatgruppen gut kontrollierbaren Reaktivität dar, während 1,4-Phenylendiamin selber einen Schmelzpunkt von über 140 °C, eine geringe Löslichkeit und gleichzeitig eine hohe, schwierig zu kontrollierende Reaktivität gegenüber Isocyanatgruppen besitzt und deshalb als Härter für Isocyanatgruppen aufweisende Zusammensetzungen kaum geeignet ist.

Es ist auch möglich, die Dialdimine der Formel (I) zusammen mit Wasser aufzubewahren, unter der Voraussetzung, dass die Dialdimine getrennt von Isocyanatgruppen gelagert werden. Dabei fallen keine Amine in fester Form aus. Erst wenn die Wasser-Aldimin-Mischung mit Isocyanatgruppen in Kontakt kommt, läuft die Hydrolyse vollständig ab. Die Reaktion zwischen Dialdiminen der Formel (I) und Isocyanatgruppen ist nämlich auch dann stark verzögert im Vergleich zur Reaktion der entsprechenden primären Amine, wenn die Aldimine zusammen mit Wasser aufbewahrt werden.

Ein weiterer Gegenstand der Erfindung ist eine härtbare Zusammensetzung, welche mindestens ein Polyisocyanat **P** und mindestens ein aromatisches Dialdimin der Formel (I) umfasst.

Der Begriff "Polyisocyanat" umfasst im vorliegenden Dokument Verbindungen mit zwei oder mehr Isocyanatgruppen, unabhängig davon, ob es sich dabei um monomere Polyisocyanate, oligomere Polyisocyanate oder Isocyanatgruppen aufweisende Polymere mit einem relativ hohen Molekulargewicht handelt.

Als Polyisocyanat **P** geeignet ist in einer Ausführungsform ein Isocyanatgruppen aufweisendes Polyurethanpolymer **PUP.**

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind PolyetherPolyurethane, Polyester-Polyurethane, Polyether-Polyharnstoffe, Polyharnstoffe, Polyester-Polyharnstoffe, Polyisocyanurate und Polycarbodiimide.

Ein geeignetes Polyurethanpolymer **PUP** ist insbesondere erhältlich aus der Umsetzung von mindestens einem Polyol mit mindestens einem Polyisocyanat. Diese Umsetzung kann dadurch erfolgen, dass das Polyol und das Polyisocyanat mit üblichen Verfahren, beispielsweise bei Temperaturen von 50 °C bis 100 °C, gegebenenfalls unter Mitverwendung geeigneter Katalysatoren, zur Reaktion gebracht werden, wobei das Polyisocyanat so dosiert ist, dass dessen Isocyanatgruppen im Verhältnis zu den Hydroxylgruppen des Polyols im stöchiometrischen Überschuss vorhanden sind. Vorteilhaft ist das Polyisocyanat so dosiert, dass ein NCO/OH-Verhältnis von 1.3 bis 5, insbesondere von 1.5 bis 3, eingehalten wird. Unter dem NCO/OH-Verhältnis wird hierbei das Verhältnis der Anzahl der eingesetzten Isocyanatgruppen zu der Anzahl der eingesetzten Hydroxylgruppen verstanden. Bevorzugt verbleibt im Polyurethanpolymer **PUP** nach der Umsetzung aller Hydroxylgruppen des Polyols ein Gehalt an freien Isocyanatgruppen von 0.5 bis 15 Gewichts-%, besonders bevorzugt von 0.5 bis 10 Gewichts-%.

Gegebenenfalls kann das Polyurethanpolymer **PUP** unter Mitverwendung von Weichmachern hergestellt werden, wobei die verwendeten Weichmacher keine gegenüber Isocyanaten reaktive Gruppen enthalten.

Als Polyole für die Herstellung eines Polyurethanpolymers **PUP** können beispielsweise die folgenden handelsüblichen Polyole oder Mischungen davon eingesetzt werden:
- Polyoxyalkylenpolyole, auch Polyetherpolyole oder Oligoetherole genannt, welche Polymerisationsprodukte von Ethylenoxid, 1,2-Propylenoxid, 1,2- oder 2,3-Butylenoxid, Oxetan, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach AST M D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.

Besonders geeignet sind Polyoxyalkylendiole oder Polyoxyalkylentriole, insbesondere Polyoxyethylen- und Polyoxypropylendi- und -triole.

Speziell geeignet sind Polyoxyalkylendiole und -triole mit einem Ungesättigtheitsgrad tiefer als 0.02 mEq/g und mit einem Molekulargewicht im Bereich von 1'000 - 30'000 g/mol, sowie Polyoxypropylendiole und -triole mit einem Molekulargewicht von 400 - 8'000 g/mol.

Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt beispielsweise aus zwei- bis dreiwertigen Alkoholen wie beispielsweise 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole mit organischen Dicarbonsäuren oder deren Anhydride oder Ester wie beispielsweise Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure und Hexahydrophthalsäure oder Mischungen der vorgenannten Säuren, sowie Polyesterpolyole aus Lactonen wie beispielsweise ε-Caprolacton.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen.
- Polyacrylat- und Polymethacrylatpolyole.
- Polykohlenwasserstoffpolyole, auch Oligohydrocarbonole genannt, wie beispielsweise polyhydroxyfunktionelle Ethylen-Propylen-, Ethylen-Butylen- oder Ethylen-Propylen-Dien-Copolymere, wie sie beispielsweise von der Firma Kraton Polymers hergestellt werden, oder polyhydroxyfunktionelle Copolymere aus Dienen wie 1,3-Butandien oder Diengemischen und Vinylmonomeren wie Styrol, Acrylnitril oder Isobutylen, oder polyhydroxyfunktionelle Polybutadienpolyole, wie beispielsweise solche, die durch Copolymerisation von 1,3-Butadien und Allylalkohol hergestellt werden und auch hydriert sein können.
- Polyhydroxyfunktionelle Acrylonitril/Butadien-Copolymere, wie sie beispielsweise aus Epoxiden oder Aminoalkoholen und carboxylterminierten Acrylonitril/Butadien-Copolymeren (kommerziell erhältlich unter dem Namen Hycar^{®} CTBN von Hanse Chemie) hergestellt werden können.

Diese genannten Polyole weisen bevorzugt ein mittleres Molekulargewicht von 250 - 30'000 g/mol, insbesondere von 400 - 20'000 g/mol, und weisen bevorzugt eine mittlere OH-Funktionalität im Bereich von 1.6 bis 3 auf.

Als Polyole bevorzugt sind Polyether-, Polyester-, Polycarbonat- und Polyacrylatpolyole, bevorzugt Di- und Triole. Besonders bevorzugt sind Polyetherpolyole, insbesondere Polyoxypropylen- und Polyoxypropylenpolyoxyethylenpolyole.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole, 1,1,1-Trimethylolethan, 1,1,1-Trimethylolpropan, Glycerin, Pentaerythrit, Zuckeralkohole wie Xylit, Sorbit oder Mannit, Zucker wie Saccharose, andere höherwertige Alkohole, niedrigmolekulare Alkoxylierungsprodukte der vorgenannten zwei- und mehrwertigen Alkohole, sowie Mischungen der vorgenannten Alkohole bei der Herstellung des Polyurethanpolymers **PUP** mitverwendet werden. Ebenso können kleine Mengen an Polyolen mit einer mittleren OH-Funktionalität von mehr als 3 mitverwendet werden, beispielsweise Zuckerpolyole.

Als Polyisocyanat für die Herstellung eines Isocyanatgruppen aufweisenden Polyurethanpolymers **PUP** werden aromatische oder aliphatische Polyisocyanate, insbesondere die Diisocyanate, eingesetzt.

Als "aromatisches Isocyanat" wird eine organische Verbindung bezeichnet, welche ausschliesslich aromatische Isocyanatgruppen aufweist. Als "aromatisch" wird eine Isocyanatgruppe bezeichnet, die an einen aromatischen oder heteroaromatischen Rest gebunden ist. Als "aliphatisches Isocyanat" wird eine organische Verbindung bezeichnet, die aliphatische Isocyanatgruppen enthält. Als "aliphatisch" wird eine Isocyanatgruppe bezeichnet, die an einen aliphatischen, cycloaliphatischen oder arylaliphatischen Rest gebunden ist.

Als aromatische Polyisocyanate eignen sich beispielsweise monomere Di- oder Triisocyanate wie 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)-benzol, Tris-(4-isocyanatophenyl)-methan, Tris-(4-isocyanatophenyl)-thiophosphat, Oligomere und Polymere der vorgenannten Isocyanate, sowie beliebige Mischungen der vorgenannten Isocyanate. Bevorzugt sind MDI und TDI.

Als aliphatische Polyisocyanate eignen sich beispielsweise monomere Di- oder Triisocyanate wie 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und -1,4-diisocyanat, 1-Methyl-2,4- und -2,6-diisocyanatocyclohexan und beliebige Gemische dieser Isomeren (HTDI oder H₆TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder H₁₂MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-lsocyanato-1-methylethyl)-naphthalin, Dimer- und Trimerfettsäureisocyanate wie 3,6-Bis-(9-isocyanatononyl)-4,5-di-(1-heptenyl)-cyclohexen (Dimeryldiisocyanat), α,α,α',α',α",α"-Hexamethyl-1,3,5-mesitylentriisocyanat, Oligomere und Polymere der vorgenannten Isocyanate, sowie beliebige Mischungen der vorgenannten Isocyanate. Bevorzugt sind HDI und IPDI.

Bevorzugt sind Polyurethanpolymere **PUP** mit aromatischen Isocyanatgruppen.

Als Polyisocyanat **P** geeignet ist in einer weiteren Ausführungsform ein Polyisocyanat **PI** in der Form eines monomeren Di- oder Triisocyanates, oder eines Oligomeren eines monomeren Diisocyanates, wobei als monomeres Di- oder Triisocyanat beispielsweise die vorgenannten aromatischen und aliphatischen Di- und Triisocyanate geeignet sind.

Als Oligomer eines monomeren Diisocyanates geeignet sind insbesondere die Oligomere von HDI, IPDI und TDI. Solche Oligomere stellen in der Praxis üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid-, Uretonimin- oder Oxadiazintrion-Gruppen. Bevorzugt weisen sie einen niedrigen Gehalt an monomeren Diisocyanaten auf. Kommerziell erhältliche Typen sind insbesondere HDI-Biurete, beispielsweise Desmodur^{®} N 100 und Desmodur^{®} N 3200 (von Bayer), Tolonate^{®} HDB und Tolonate^{®} HDB-LV (von Rhodia) sowie Duranate^{®} 24A-100 (von Asahi Kasei); HDI-Isocyanurate, beispielsweise Desmodur^{®} N 3300, Desmodur^{®} N 3600 und Desmodur^{®} N 3790 BA (von Bayer), Tolonate^{®} HDT, Tolonate^{®} HDT-LV und Tolonate^{®} HDT-LV2 (von Rhodia), Duranate^{®} TPA-100 und Duranate^{®} THA-100 (von Asahi Kasei) sowie Coronate^{®} HX (von Nippon Polyurethane); HDI-Uretdione, beispielsweise Desmodur^{®} N 3400 (von Bayer); HDI-Iminooxadiazindione, beispielsweise Desmodur^{®} XP 2410 (von Bayer); HDI-Allophanate, beispielsweise Desmodur^{®} VP LS 2102 (von Bayer); IPDI-Isocyanurate, beispielsweise Desmodur^{®} Z 4470 (von Bayer) und Vestanat^{®} T1890/100 (von Evonik); TDI-Oligomere, beispielsweise Desmodur^{®} IL (von Bayer); sowie gemischte Isocyanurate auf Basis TDI / HDI, zum Beispiel Desmodur^{®} HL (von Bayer).

In einer weiteren Ausführungsform stellt das Polyisocyanat P ein Polyisocyanat **PI** in der Form einer bei Raumtemperatur flüssigen Form von MDI oder einer bei Raumtemperatur flüssigen Form von polymerem MDI (PMDI) dar.

Bei Raumtemperatur flüssige Formen von MDI (sogenanntes "modifiziertes MDI") stellen Gemische von MDI mit MDI-Derivaten, wie beispielsweise MDI-Carbodiimiden, MDI-Uretoniminen oder MDI-Urethanen, dar. Kommerziell erhältliche Typen von modifiziertem MDI sind beispielsweise Desmodur^{®} CD, Desmodur^{®} PF und Desmodur^{®} PC (von Bayer), Lupranat^{®} MM 103 (von BASF), Isonate^{®} M 143 (von Dow) sowie Suprasec^{®} 2020 und Suprasec^{®} 2388 (von Huntsman).

Als polymeres MDI oder PMDI werden Gemische aus MDI und MDI-Homologen bezeichnet. Kommerziell erhältliche Typen von PMDI sind beispielsweise Desmodur^{®} VL, Desmodur^{®} VL 50, Desmodur^{®} VL R 10, Desmodur^{®} VL R 20 und Desmodur^{®} VKS 20 F (von Bayer), Lupranat^{®} M 10 R und Lupranat^{®} M 20 R (von BASF), Isonate^{®} M 309, Voranate^{®} M 229 und Voranate M^{®} 580 (von Dow) sowie Suprasec^{®} 5025, Suprasec^{®} 2050 und Suprasec^{®} 2487 (von Huntsman).

Als Polyisocyanat **PI** bevorzugt sind PMDI, bei Raumtemperatur flüssige Formen von MDI, sowie die Oligomeren von HDI, IPDI und TDI, insbesondere die Isocyanurate.

Als Polyisocyanat **PI** besonders bevorzugt sind solche mit aromatischen Isocyanatgruppen.

Als Polyisocyanat **PI** am meisten bevorzugt sind MDI-Typen, insbesondere solche mit hohem Anteil an 2,4'-Isomer, weiterhin PMDI, sowie weiterhin bei Raumtemperatur flüssige Formen von MDI.

In noch einer weiteren Ausführungsform ist das Polyisocyanat **P** eine Mischung bestehend aus mindestens einem Polyurethanpolymer **PUP** und mindestens einem Polyisocyanat **PI,** wie sie vorgängig beschrieben wurden.

Üblicherweise ist das Polyisocyanat **P** in einer Menge von 5 bis 95 Gewichts-%, bevorzugt in einer Menge von 10 bis 90 Gewichts-%, bezogen auf die gesamte Zusammensetzung, vorhanden. In gefüllten Zusammensetzungen, d.h. Zusammensetzungen, welche einen Füllstoff enthalten, ist das Polyisocyanat **P** bevorzugt in einer Menge von 5 bis 60 Gewichts-%, insbesondere 10 bis 50 Gewichts-%, bezogen auf die gesamte Zusammensetzung, vorhanden.

Die härtbare Zusammensetzung enthält neben mindestens einem Polyisocyanat **P** mindestens ein aromatisches Dialdimin der Formel (I), wie es vorgängig detailliert beschrieben wurde, respektive eine bevorzugte Ausführungsform davon.

Das Dialdimin der Formel (I) ist in der härtbaren Zusammensetzung bevorzugt in einer leicht überstöchiometrischen, stöchiometrischen oder unterstöchiometrischen Menge, bezogen auf die Isocyanatgruppen, vorhanden. Vorteilhaft ist das Dialdimin der Formel (I) in einer derartigen Menge in der Zusammensetzung vorhanden, dass das Verhältnis zwischen der Anzahl der Aldiminogruppen und der Anzahl der Isocyanatgruppen 0.1 bis 1.1, insbesondere 0.15 bis 1.0, besonders bevorzugt 0.2 bis 0.9, beträgt.

Als Dialdimin der Formel (I) können auch Mischungen verschiedener Dialdimine der Formel (I) verwendet werden. Insbesondere können Mischungen verschiedener Dialdimine eingesetzt werden, welche ausgehend von Mischungen verschiedener Diamine **B** der Formel (II) und/oder Mischungen verschiedener Aldehyde **ALD** der Formel (III) hergestellt sind.

Die härtbare Zusammensetzung kann zusätzlich zu mindestens einem Polyisocyanat **P** und zu mindestens einem aromatischen Dialdimin der Formel (I) weitere Hilfs- und Zusatzstoffe enthalten.

Die härtbare Zusammensetzung reagiert mit Wasser bzw. Feuchtigkeit und wird dadurch vernetzt. Ist genügend Wasser vorhanden, um einen Grossteil oder alle Isocyanatgruppen umzusetzen, entsteht eine ausgehärtete Zusammensetzung, die gute mechanische Eigenschaften aufweist. Die Zusammensetzung wird deshalb als "feuchtigkeitshärtend" bezeichnet.

Die härtbare Zusammensetzung kann in der Form einer einkomponentigen Zusammensetzung oder in der Form einer zweikomponentigen Zusammensetzung vorliegen.

Als "einkomponentige Zusammensetzung" wird im vorliegenden Dokument eine härtbare Zusammensetzung bezeichnet, deren Bestandteile in vermischter Form im gleichen Gebinde gelagert werden, und welche bei Raumtemperatur über einen längeren Zeitraum lagerstabil ist, sich also in ihren Anwendungs- oder Gebrauchseigenschaften durch die Lagerung nicht oder nur unwesentlich verändert, und welche nach der Applikation durch die Einwirkung von Feuchtigkeit aushärtet.

Als "zweikomponentige Zusammensetzung" wird im vorliegenden Dokument eine härtbare Zusammensetzung bezeichnet, deren Bestandteile in zwei verschiedenen Komponenten vorliegen, die in voneinander getrennten Gebinden gelagert werden und jeweils für sich lagerstabil sind. Erst kurz vor oder während der Applikation der Zusammensetzung werden die beiden Komponenten miteinander vermischt, worauf die vermischte Zusammensetzung aushärtet, wobei die Aushärtung unter Umständen erst durch die Einwirkung von Feuchtigkeit abläuft oder vervollständigt wird.

Einkomponentige Zusammensetzungen weisen den Vorteil auf, dass sie ohne Mischvorgang applizierbar sind, während zweikomponentige Zusammensetzungen den Vorteil aufweisen, dass sie rascher aushärten und als Bestandteile Substanzen enthalten können, welche zusammen mit Isocyanaten nicht lagerfähig sind.

In einer Ausführungsform liegt die härtbare Zusammensetzung in der Form einer einkomponentigen Zusammensetzung vor.

Als Polyisocyanat **P** ist in der einkomponentigen Zusammensetzung ein Polyurethanpolymer **PUP,** oder eine Mischung aus einem Polyurethanpolymer **PUP** und einem Polyisocyanat **PI,** wie sie vorgängig beschrieben wurden, bevorzugt.

Als Hilfs- und Zusatzstoffe für eine einkomponentige Zusammensetzung sind beispielsweise die folgenden Substanzen geeignet:
- Weichmacher, beispielsweise Carbonsäureester wie Phthalate, beispielsweise Dioctylphthalat, Diisononylphthalat oder Diisodecylphthalat, Adipate, beispielsweise Dioctyladipat, Azelate und Sebacate, organische Phosphor- und Sulfonsäureester oder Polybutene;
- nicht-reaktive thermoplastische Polymere, wie beispielsweise Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere Polyethylene (PE), Polypropylene (PP), Polyisobutylene, Ethylenvinylacetat-Copolymere (EVA) und ataktische Poly-α-Olefine (APAO);
- Lösemittel;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (BaSO₄, auch Schwerspat genannt), Quarzmehle, calcinierte Kaoline, Aluminiumoxide, Aluminium-hydroxide, Kieselsäuren, insbesondere hochdisperse Kieselsäuren aus Pyrolyseprozessen, Russe, insbesondere industriell hergestellte Russe (im Folgenden als "Russ" bezeichnet), PVC-Pulver oder Hohlkugeln;
- Fasern, beispielsweise aus Polyethylen;
- Pigmente, beispielsweise Titandioxid oder Eisenoxide;
- Katalysatoren, welche die Hydrolyse der Aldiminogruppen beschleunigen, insbesondere Säuren oder zu Säuren hydrolysierbare Verbindungen, beispielsweise organische Carbonsäuren wie Benzoesäure, Salicylsäure oder 2-Nitrobenzoesäure, organische Carbonsäureanhydride wie Phthalsäureanhydrid, Hexahydrophthalsäureanhydrid und Hexahydromethylphthalsäureanhydrid, Silylester von organischen Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester;
- Katalysatoren, welche die Reaktion der Isocyanatgruppen mit Wasser beschleunigen, insbesondere Metallverbindungen, beispielsweise Organozinnverbindungen wie Dibutylzinndiacetat, Dibutylzinndilaurat, Dibutylzinndichlorid, Dibutylzinndiacetylacetonat und Dioctylzinndilaurat, Bismutverbindungen wie Bismuttrioctoat und Bismuttris(neodecanoat), und tertiäre Aminogruppen enthaltende Verbindungen wie 2,2'-Dimorpholinodiethylether und 1,4-Diazabicyclo[2.2.2]octan;
- Rheologie-Modifizierer wie beispielsweise Verdickungsmittel oder Thixotropiermittel, zum Beispiel Harnstoffverbindungen, Polyamidwachse, Bentonite oder pyrogene Kieselsäuren;
- Reaktivverdünner und Vernetzer, beispielsweise monomere Diisocyanate sowie Oligomere und Derivate dieser Diisocyanate, Addukte monomerer Diisocyanate mit kurzkettigen Polyolen, sowie Adipinsäuredihydrazid und andere Dihydrazide, sowie blockierte Amine in Form von Ketiminen, Oxazolidinen, Enaminen oder nicht der Formel (I) entsprechenden Aldiminen, die erhältlich sind ausgehend von anderen Aminen als den aromatischen Diaminen **B** der Formel (II) und/oder anderen Aldehyden als den Aldehyden **ALD** der Formel (III), insbesondere die in WO 2004/013088 A1 beschriebenen Polyaldimine und insbesondere Polyaldimine ausgehend von oligomeren Formen von Diaminodiphenylmethan (PMDA);
- Trocknungsmittel, wie beispielsweise Molekularsiebe, Calciumoxid, hochreaktive Isocyanate wie p-Tosylisocyanat, Orthoameisensäureester, Tetraalkoxysilane wie Tetraethoxysilan;
- Organoalkoxysilane, im Folgenden auch "Silane" genannt, wie beispielsweise Epoxysilane, (Meth)acrylsilane, Isocyanatosilane, Vinylsilane, Carbamatosilane, Alkylsilane, S-(Alkylcarbonyl)-mercaptosilane und Aldiminosilane, sowie oligomere Formen dieser Silane;
- Stabilisatoren gegen Wärme, Licht- und UV-Strahlung;
- flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen.

Es ist vorteilhaft, darauf zu achten, dass solche Zusätze die Lagerstabilität der Zusammensetzung nicht beeinträchtigen. Das heisst, dass diese Zusätze während der Lagerung die zur Vernetzung führenden Reaktionen wie Hydrolyse der Aldiminogruppen oder Vernetzung der Isocyanatgruppen nicht in signifikantem Ausmass auslösen dürfen. Insbesondere bedeutet dies, dass all diese Zusätze kein oder höchstens Spuren von Wasser enthalten sollten. Es kann deshalb sinnvoll sein, gewisse Zusätze vor dem Einmischen in die Zusammensetzung chemisch oder physikalisch zu trocknen.

Bevorzugt enthält die einkomponentige Zusammensetzung neben mindestens einem Polyisocyanat **P** und mindestens einem aromatischen Dialdimin der Formel (I) mindestens einen Katalysator. Insbesondere enthält die Zusammensetzung als Katalysator eine Carbonsäure wie Benzoesäure oder Salicylsäure und/oder eine Zinnverbindung und/oder eine Bismutverbindung. Es kann vorteilhaft sein, wenn unterschiedliche Katalysatoren, bzw. unterschiedliche Katalysatorenarten wie beispielsweise eine Säure und eine Metallverbindung, miteinander gemischt werden.

Weiterhin bevorzugt enthält die einkomponentige Zusammensetzung neben mindestens einem Polyisocyanat **P** und mindestens einem aromatischen Dialdimin der Formel (I) mindestens einen weiteren Hilfs- und Zusatzstoff, insbesondere ausgewählt aus der Gruppe umfassend Weichmacher, Vernetzer, Füllstoffe und Verdickungsmittel.

Die beschriebene einkomponentige Zusammensetzung wird vorzugsweise unter Ausschluss von Feuchtigkeit hergestellt und aufbewahrt. In einer geeigneten klimadichten Verpackung oder Anordnung, wie beispielsweise einem Fass, einem Beutel oder einer Kartusche, verfügt sie über eine hervorragende Lagerstabilität. Mit den Begriffen "lagerstabil" und "Lagerstabilität" in Zusammenhang mit einer härtbaren Zusammensetzung wird im vorliegenden Dokument der Sachverhalt bezeichnet, dass die Viskosität der Zusammensetzung bei gegebener Applikationstemperatur und bei geeigneter Lagerung in der betrachteten Zeitspanne nicht oder höchstens so stark ansteigt, dass die Zusammensetzung auf die vorgesehene Weise verwendbar bleibt.

Kommt die beschriebene einkomponentige Zusammensetzung in Kontakt mit Feuchtigkeit bzw. Wasser, beginnen die Aldiminogruppen des Dialdimins der Formel (I) zu hydrolysieren. Die in der Zusammensetzung vorhandenen Isocyanatgruppen reagieren darauf mit den hydrolysierenden Aldiminogruppen, wobei der Aldehyd **ALD** der Formel (III) freigesetzt wird. Im Verhältnis zu den Aldiminogruppen überschüssige Isocyanatgruppen reagieren direkt mit Wasser. Als Ergebnis dieser Reaktionen vernetzt die Zusammensetzung und härtet schliesslich zu einem festen Material aus. Die Reaktion der Isocyanatgruppen mit den hydrolysierenden Aldiminogruppen muss dabei nicht notwendigerweise über freie Aminogruppen erfolgen; es sind auch Reaktionen mit Zwischenstufen der Hydrolysereaktion möglich. Beispielsweise ist es denkbar, dass eine hydrolysierende Aldiminogruppe in der Form eines Halbaminals direkt mit einer Isocyanatgruppe reagiert.

Das für die Aushärtungsreaktion benötigte Wasser kann entweder aus der Luft stammen (Luftfeuchtigkeit), oder aber die Zusammensetzung kann mit einer Wasser enthaltenden Komponente in Kontakt gebracht werden, zum Beispiel durch Besprühen, oder es kann der Zusammensetzung bei der Applikation eine Wasser enthaltende Komponente zugesetzt werden, insbesondere durch Einmischen.

Die Aushärtung erfolgt im Allgemeinen blasenfrei, insbesondere auch bei hoher Aushärtungsgeschwindigkeit.

Die Aushärtungsgeschwindigkeit lässt sich über die Art und Menge eines oder mehrerer gegebenenfalls vorhandener Katalysatoren, über die bei der Aushärtung herrschende Temperatur sowie über die Luftfeuchtigkeit bzw. die Menge zugesetzten Wassers beeinflussen. Wird der Zusammensetzung eine Wasser enthaltene Komponente zugesetzt, erfolgt die Aushärtung stark beschleunigt im Vergleich zur Aushärtung ausschliesslich mit Luftfeuchtigkeit. Somit beschreibt die vorliegende Erfindung auch eine ausgehärtete Zusammensetzung.

In einer weiteren Ausführungsform liegt die härtbare Zusammensetzung in der Form einer zweikomponentigen Zusammensetzung vor. Die zweikomponentige Zusammensetzung besteht aus einer Komponente **K1** und einer Komponente **K2,** welche getrennt voneinander gelagert und erst kurz vor der Applikation miteinander vermischt werden.

In einer Ausführungsform der zweikomponentigen Zusammensetzung sind das Polyisocyanat **P** und das Dialdimin der Formel (I) Teil der Komponente **K1,** während die Komponente **K2** gegenüber Isocyanatgruppen reaktive Verbindungen, insbesondere Wasser und/oder Polyole und/oder Polyamine und/oder Aminoalkohole und/oder Polythiole enthält.

In einer anderen Ausführungsform der zweikomponentigen Zusammensetzung ist das Polyisocyanat **P** Teil der Komponente **K1,** während die Komponente **K2** das Dialdimin der Formel (I) sowie gegenüber Isocyanatgruppen reaktive Verbindungen, insbesondere Wasser und/oder Polyole und/oder Polyamine und/oder Aminoalkohole und/oder Polythiole, enthält.

Bevorzugt enthält die Komponente **K2** mindestens ein Dialdimin der Formel (I) und Wasser.

Als Polyisocyanat **P** ist in der zweikomponentigen Zusammensetzung ein Polyisocyanat **PI,** oder eine Mischung aus einem Polyurethanpolymer **PUP** und einem Polyisocyanat **PI,** wie sie vorgängig beschrieben wurden, bevorzugt.

Als Polyole in der Komponente **K2** geeignet sind dieselben handelsüblichen Polyole, wie sie bereits als geeignet zur Herstellung eines Polyurethanpolymers **PUP** erwähnt wurden, sowie diejenigen niedrigmolekularen zwei- oder mehrwertigen Alkohole, wie sie vorgängig als geeignet zum Mitverwenden bei der Herstellung eines Polyurethanpolymers **PUP** erwähnt wurden. Als Polyamine in der Komponente **K2** geeignet sind handelsübliche aliphatische oder aromatische Polyamine mit primären und/ oder sekundären Aminogruppen, wie sie üblicherweise in zweikomponentigen Polyurethanzusammensetzungen eingesetzt werden, wie beispielsweise 1,5-Diamino-2-methylpentan (MPMD), 1,3-Xylylendiamin (MXDA), N,N'-Dibutyl-ethylendiamin, 3,5-Diethyl-2,4(6)-diaminotoluol (DETDA), 3,5-Dimethylthio-2,4(6)-diaminotoluol (Ethacure^{®} 300, Albemarle) sowie primäre und sekundäre Polyoxyalkylen-Diamine, wie sie zum Beispiel unter dem Namen Jeffamine^{®} (von Huntsman Chemicals) erhältlich sind. Als Aminoalkohole in der Komponente **K2** geeignet sind Verbindungen, welche mindestens eine primäre oder sekundäre Aminogruppe und mindestens eine Hydroxylgruppe aufweisen, wie beispielsweise 2-Aminoethanol, 2-Methylaminoethanol, 1-Amino-2-propanol und Diethanolamin. Als Polythiole in der Komponente **K2** geeignet sind beispielsweise unter dem Markennamen Thiokol^{®} bekannte flüssige Mercapto-terminierte Polymere, sowie Polyester von Thiocarbonsäuren. Falls die Komponente **K2** Wasser enthält, ist es vorteilhaft, wenn die Menge des Wassers höchstens der zur Hydrolyse des Dialdimins - und gegebenenfalls vorhandener weiterer blockierter Amine - benötigten Menge Wasser entspricht. Zudem können beide Komponenten weitere Hilfs- und Zusatzstoffe, wie sie bereits vorgängig für eine einkomponentige Zusammensetzung erwähnt wurden, enthalten. Im Falle der Komponente **K2** sind jedoch zusätzlich noch weitere Hilfs- und Zusatzstoffe möglich. Insbesondere sind dies solche Hilfs- und Zusatzstoffe, welche zusammen mit aromatischen Isocyanatgruppen nicht oder nur kurzzeitig lagerfähig sind. Insbesondere sind dies Katalysatoren wie:
Verbindungen von Zink, Mangan, Eisen, Chrom, Cobalt, Kupfer, Nickel, Molybdän, Blei, Cadmium, Quecksilber, Antimon, Vanadium, Titan, Zirconium oder Kalium, wie Zink(II)-acetat, Zink(II)-2-ethylhexanoat, Zink(II)-laurat, Zink(II)-oleat, Zink(II)-naphthenat, Zink(II)-acetylacetonat, Zink(II)-salicylat, Mangan(II)-2-ethylhexanoat, Eisen(III)-2-ethylhexanoat, Eisen(III)-acetylacetonat, Chrom(III)-2-ethylhexanoat, Cobalt(II)-naphthenat, Cobalt(II)-2-ethylhexanoat, Kupfer(II)-2-ethylhexanoat, Nickel(II)-naphthenat, Phenylquecksilber-neodecanoat, Blei(II)-acetat, Blei(II)-2-ethylhexanoat, Blei(II)-neodecanoat, Blei(II)-acetylacetonat, Aluminiumlactat, Aluminiumoleat, Aluminium(III)-acetylacetonat, Diisopropoxytitan-bis-(ethylacetoacetat), Dibutoxytitan-bis-(ethylacetoacetat), Dibutoxytitan-bis-(acetylacetonat), Kalium-acetat, Kaliumoctoat; tertiäre Amine wie Triethylamin, Tributylamin, N-Ethyl-diisopropylamin, N,N,N',N'-Tetramethyl-ethylendiamin, Pentamethyl-diethylentriamin und höhere Homologe davon, N,N,N',N'-Tetramethyl-propylendiamin, Pentamethyl-dipropylentriamin und höhere Homologe davon, N,N,N',N'-Tetramethyl-1,3-butandi-amin, N,N,N',N'-Tetramethyl-1,6-hexandiamin, Bis-(dimethylamino)-methan, N,N-Dimethylbenzylamin, N,N-Dimethylcyclohexylamin, N-Methyl-dicyclohexylamin, N,N-Dimethyl-hexadecylamin, Bis-(N,N-diethylaminoethyl)-adipat, N,N-Dimethyl-2-phenylethylamin, Tris-(3-dimethylaminopropyl)-amin, 1,4-Diazabicyclo[2.2.2]octan (DABCO), 1,8-Diazabicyclo[5.4.0]undec-7-en (DBU), 1,5-Diazabicyclo[4.3.0]non-5-en (DBN), N-Methylmorpholin, N-Ethylmorpholin, N-Cocomorpholin, N,N'-Dimethylpiperazin, N-Methyl-N'-dimethylaminoethyl-piperazin, Bis-(dimethylaminoethyl)-piperazin, 1,3,5-Tris-(dimethylaminopropyl)-hexahydrotriazin, Bis-(2-dimethylaminoethyl)-ether; stickstoffaromatische Verbindungen wie 4-Dimethylamino-pyridin, N-Methylimidazol, N-Vinylimidazol oder 1,2-Dimethylimidazol; Amidine und Guanidine wie 1,1,3,3-Tetramethylguanidin; tertiäre Amine enthaltend aktive Wasserstoffatome, wie Triethanolamin, Triisopropanolamin, N-Methyldiethanolamin, N,N-Dimethylethanolamin, 3-(Dimethylamino)-propyl-diisopropanolamin, Bis-(3-(dimethylamino)-propyl)-isopropanolamin, Bis-(3-dimethylaminopropyl)amin, 3-(Dimethylamino)-propyl-harnstoff, Mannich-Basen wie 2,4,6-Tris-(dimethylaminomethyl)-phenol oder 2,4,6-Tris-(3-(dimethylamino)-propylaminomethyl)-phenol, N-Hydroxypropylimidazol, N-(3-Aminopropyl)-imidazol, sowie Alkoxylierungs- und Polyalkoxylierungsprodukte dieser Verbindungen, beispielsweise Dimethylaminoethoxyethanol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen, wie Umsetzungsprodukte aus tertiären Aminen und Carbonsäuren oder Phenolen, beispielsweise aus 1,4-Diazabicyclo[2.2.2]octan oder DBU und Ameisensäure oder Essigsäure; sowie Kombinationen der genannten Verbindungen, insbesondere von Metallverbindungen und tertiären Aminen.

Bevorzugt enthält die zweikomponentige Zusammensetzung neben mindestens einem Polyisocyanat **P** und mindestens einem aromatischen Dialdimin der Formel (I) mindestens einen Katalysator. Insbesondere enthält die Zusammensetzung als Katalysator eine Carbonsäure wie Benzoesäure oder Salicylsäure und/oder eine Zinnverbindung und/oder eine Bismutverbindung. Es kann vorteilhaft sein, wenn unterschiedliche Katalysatoren, bzw. unterschiedliche Katalysatorenarten wie beispielsweise eine Säure und eine Metallverbindung, miteinander gemischt werden.

Weiterhin bevorzugt enthält die zweikomponentige Zusammensetzung neben mindestens einem Polyisocyanat **P** und mindestens einem aromatischen Dialdimin der Formel (I) mindestens einen weiteren Hilfs- und Zusatzstoff, insbesondere ausgewählt aus der Gruppe umfassend Weichmacher, Vernetzer, Füllstoffe und Verdickungsmittel.

Bevorzugt enthält die Komponente **K2** keine Isocyanatgruppen.

Falls die zweikomponentige Zusammensetzung weitere blockierte Amine, insbesondere Ketimine, Oxazolidine, Enamine oder nicht der Formel (I) entsprechende Aldimine, enthält, können diese Teil der Komponente **K1** und/oder **K2** sein; bevorzugt sind sie Teil der Komponente **K2.** Die blockierten Amine haben die Eigenschaft, bei ihrer Hydrolyse Aminogruppen freizusetzen, welche schnell mit vorhandenen Isocyanatgruppen reagieren.

Die Herstellung der beschriebenen Komponenten **K1** und **K2** erfolgt getrennt voneinander, mindestens für die Komponente **K1** unter Ausschluss von Feuchtigkeit. Die beiden Komponenten **K1** und **K2** sind getrennt voneinander lagerstabil, d.h. sie können jede in einer geeigneten Verpackung oder Anordnung, wie beispielsweise in einem Fass, einem Hobbock, einem Beutel, einem Eimer oder einer Kartusche, vor ihrer Anwendung während mehreren Monaten bis zu einem Jahr und länger aufbewahrt werden, ohne dass sie sich in ihren jeweiligen Eigenschaften in einem für ihren Gebrauch relevanten Ausmass verändern.

Das Mischungsverhältnis zwischen den Komponenten **K1** und **K2** wird bevorzugt so gewählt, dass die gegenüber Isocyanatgruppen reaktiven Gruppen der Komponenten **K1** und **K2** in einem geeigneten Verhältnis zu den Isocyanatgruppen der Komponente **K1** stehen. In der beschriebenen zweikomponentigen Zusammensetzung sind vor der Aushärtung geeigneterweise 0.1 bis 1.1, bevorzugt 0.5 bis 0.95, besonders bevorzugt 0.6 bis 0.95 Equivalent der Summe der gegenüber Isocyanaten reaktiven Gruppen pro Equivalent Isocyanatgruppen vorhanden, wobei die Aldiminogruppen und gegebenenfalls vorhandene weitere blockierte Aminogruppen zu den gegenüber Isocyanaten reaktiven Gruppen gezählt werden, und Wasser nicht zu den gegenüber Isocyanaten reaktiven Gruppen gerechnet wird. Überschüssige Isocyanatgruppen reagieren insbesondere direkt mit Wasser, beispielsweise mit Luftfeuchtigkeit.

Vor oder während der Applikation der beschriebenen zweikomponentigen Zusammensetzung werden die Komponenten **K1** und **K2** mittels eines geeigneten Verfahrens miteinander vermischt. Das Mischen kann kontinuierlich oder batchweise erfolgen. Die vermischte Zusammensetzung wird während dem Mischen oder anschliessend an das Mischen appliziert, indem sie mit einer Festkörperoberfläche kontaktiert wird, gegebenenfalls mittels eines geeigneten Hilfsmittels. Dabei muss darauf geachtet werden, dass zwischen dem Vermischen der Komponenten **K1** und **K2** und der Applikation nicht zuviel Zeit vergeht, da es dadurch zu Störungen, wie beispielsweise einem verlangsamten oder unvollständigen Aufbau der Haftung zur Festkörperoberfläche, kommen kann. Die maximale Zeitspanne, innerhalb welcher die vermischte Zusammensetzung appliziert sein sollte, wird als "Topfzeit" oder auch als "Offenzeit" bezeichnet. Oft wird als Offenzeit die Zeit definiert, in welcher sich die Viskosität der vermischten Zusammensetzung verdoppelt.

Nach dem Vermischen der Komponenten **K1** und **K2** beginnt die Aushärtung. Das Dialdimin der Formel (I) beginnt in der bereits beschriebenen Weise zu hydrolysieren und mit den Isocyanatgruppen zu reagieren, sobald es mit Wasser in Kontakt kommt. Entweder ist das Wasser in der vermischten Zusammensetzung bereits vorhanden - indem es ein Bestandteil der Komponente **K2** war, oder indem es der Zusammensetzung vor oder während dem Vermischen der Komponenten **K1** und **K2** zugesetzt wurde -, oder das Wasser diffundiert in der Form von Luftfeuchtigkeit in die vermischte Zusammensetzung. Im letztgenannten Fall erfolgt die Reaktion des Dialdimins mit den Isocyanatgruppen von aussen nach innen, parallel zum Eindringen der Luftfeuchtigkeit in die Zusammensetzung. Wie bereits beschrieben, muss die Reaktion der Isocyanatgruppen mit den hydrolysierenden Aldiminogruppen nicht notwendigerweise über freie Aminogruppen erfolgen, sondern kann auch über Zwischenstufen der Hydrolysereaktion erfolgen. Auf die gleiche Weise werden die Reaktivgruppen von weiteren gegebenenfalls in der Zusammensetzung vorhandenen latenten Härtern freigesetzt. Weiterhin reagieren nach dem Vermischen der Komponenten **K1** und **K2** die in der Zusammensetzung gegebenenfalls vorhandenen gegenüber Isocyanatgruppen reaktiven Verbindungen, wie insbesondere Polyole und Polyamine, mit den Isocyanatgruppen. Überschüssige Isocyanatgruppen reagieren insbesondere direkt mit Wasser. Als Ergebnis dieser Reaktionen vernetzt die vermischte Zusammensetzung und härtet schliesslich zu einem festen Material aus. Somit beschreibt die vorliegende Erfindung auch eine ausgehärtete Zusammensetzung.

Die Aushärtung erfolgt im Allgemeinen blasenfrei, insbesondere auch bei hoher Aushärtungsgeschwindigkeit.

Die Aushärtungsgeschwindigkeit lässt sich über die Art und Menge eines oder mehrerer gegebenenfalls vorhandener Katalysatoren, über die bei der Aushärtung herrschende Temperatur sowie über die Luftfeuchtigkeit bzw. die Menge des über die Komponente **K2** eingebrachten Wassers beeinflussen.

Die beschriebenen härtbaren Zusammensetzungen enthaltend mindestens ein Polyisocyanat **P** und mindestens ein aromatisches Dialdimin der Formel (I) weisen eine ganze Reihe von vorteilhaften Eigenschaften auf.

Sie sind in einem einfachen Verfahren herstellbar, insbesondere aufgrund der Tatsache, dass die Dialdimine der Formel (I) bei Raumtemperatur flüssig sind. Als Dialdimine der Formel (I) liegen nun auch hochschmelzende primäre aromatische Diamine, wie insbesondere 1,4-Phenylendiamin, in einer für den Einsatz in einer härtbaren Zusammensetzung geeigneten Form vor, welche bisher in bei Raumtemperatur verarbeiteten, insbesondere lösemittelfreien Polyurethanzusammensetzungen nur sehr schwierig oder gar nicht einsetzbar waren. Härtbare Zusammensetzungen, welche niedrigviskose Diadimine der Formel (I) enthalten, weisen eine vorteilhafte Konsistenz mit einer relativ tiefen Grundviskosität auf, welche für viele Anwendungen eine gute Verarbeitbarkeit der Zusammensetzung ermöglicht.

Weiterhin weisen die beschriebenen härtbaren Zusammensetzungen eine gute Lagerstabilität auf, insbesondere auch dann, wenn die Dialdimine der Formel (I) zusammen mit dem Polyisocyanat **P** aufbewahrt werden. Auch für den bevorzugten Fall, das das Polyisocyanat **P** aromatische Isocyanatgruppen aufweist, ist die Lagerstabilität zusammen mit Dialdiminen der Formel (I) gut.

Weiterhin weisen die beschriebenen härtbaren Zusammensetzungen vor, während und nach der Aushärtung nur einen sehr geringen Geruch auf, oder sind ganz geruchsfrei, was beim Vorhandensein von Aldehyden im Allgemeinen nicht der Fall ist. Zusammensetzungen, welche als Dialdimine der Formel (I) ausschliesslich die bevorzugten Dialdimine abgeleitet von Aldehyden **ALD1** der Formel (III a) enthalten, sind vor, während und nach der Aushärtung geruchsfrei; selbstverständlich unter der Voraussetzung, dass sie keine anderen Bestandteile mit einem starken Geruch enthalten.

Weiterhin weisen die beschriebenen härtbaren Zusammensetzungen, insbesondere in Form von einkomponentigen Zusammensetzungen, eine relativ lange Offenzeit auf. Überraschenderweise ist die Aushärtungsgeschwindigkeit dieser Zusammensetzungen aber trotz der relativ langen Offenzeit nicht länger als beispielsweise die Aushärtungsgeschwindigkeit von entsprechenden Zusammensetzungen enthaltend aliphatische Dialdimine, welche ausgehend von aliphatischen Aminen hergestellt sind. Die Kombination aus langer Offenzeit und rascher Aushärtung ist in der Praxis für viele einkomponentige und zweikomponentige Anwendungen äusserst erwünscht. So ist die Anwendung einer einkomponentigen Zusammensetzung oft einfacher, wenn nach deren Applikation noch etwas Zeit bleibt, um die Zusammensetzung in die erwünschte Form zu bringen, bevor sich eine Haut aus angehärtetem Material auf der Oberfläche gebildet hat. Auch die Anwendung einer zweikomponentigen Zusammensetzung ist oft wesentlich einfacher, wenn diese eine verlängerte Offenzeit aufweist, müssen doch sowohl der Mischvorgang als auch die Applikation der Zusammensetzung, und gegebenenfalls notwendige Nachbearbeitungsschritte, beispielsweise um die Zusammensetzung in die erwünschte Form zu bringen, innerhalb der Offenzeit erfolgen. Anschliessend soll die Zusammensetzung aber rasch aushärten, um möglichst bald belastbar zu sein. Für den Fall, dass eine beschriebene einkomponentige Zusammensetzung mit einer Wasser enthaltenen Komponente beschleunigt ausgehärtet wird, wirkt sich die relativ lange Offenzeit besonders vorteilhaft aus, da in solchen beschleunigten Systemen das für die Applikation zur Verfügung stehende Zeitfenster in der Regel sehr knapp bemessen ist.

Die beschriebenen härtbaren Zusammensetzungen härten im Allgemeinen ohne die Bildung von Blasen aus. Dies gilt auch bei hoher Aushärtungsgeschwindigkeit.

Weiterhin weisen die beschriebenen härtbaren Zusammensetzungen im ausgehärteten Zustand gute mechanische Eigenschaften und eine gute Beständigkeit gegenüber Wärme und Feuchtigkeit auf. Die bevorzugten Zusammensetzungen enthaltend ein aromatisches Polyisocyanat **P** weisen eine besonders gute Beständigkeit gegenüber Wärme und Feuchtigkeit sowie besonders gute mechanische Eigenschaften auf, insbesondere eine hohe Festigkeit bei einer hohen Dehnbarkeit. Die gute Wärmestabilität kann möglicherweise auf die Tatsache zurückgeführt werden, dass die bei der Aushärtungsreaktion der aromatischen Isocyanatgruppen mit den aromatischen Aldiminogruppen entstehenden Harnstoffgruppen beidseitig aromatisch substituiert sind und deshalb keine Neigung zum Austausch der Substituenten zeigen.

Härtbare Zusammensetzungen umfassend mindestens ein Polyisocyanat **P** und mindestens eines der besonders bevorzugten Dialdimine der Formel (I) ausgehend von 1,4-Phenylendiamin und einem Aldehyd **ALD1** der Formel (III a) weisen besonders vorteilhafte Eigenschaften auf. Sie sind besonders einfach herzustellen aufgrund der Tatsache, dass es sich bei diesen Dialdiminen der Formel (I) um geruchsfreie, niedrigviskose Flüssigkeiten handelt, und sie weisen im ausgehärteten Zustand besonders gute mechanische Eigenschaften, insbesondere besonders hohe Festigkeiten, auf, insbesondere wenn das Polyisocyanat **P** ein aromatisches Polyisocyanat **P** ist. Ein weiterer Vorteil dieser Zusammensetzungen besteht darin, dass die von 1,4-Phenylendiamin abgeleiteten Aldimine der Formel (I) eine für aromatische Amine vergleichsweise hohe Reaktivität gegenüber Isocyanaten aufweisen, was zu einer vergleichsweise kurzen Hautbildungszeit der Zusammensetzungen führt. Ein Vorteil besteht schliesslich auch darin, dass das dem Dialdimin zugrunde liegende 1,4-Phenylendiamin eine für aromatische Amine niedrige Toxizität aufweist, was beispielsweise darin zum Ausdruck kommt, dass es weder als mutagen noch als karzinogen eingestuft ist.

Mit den erfindungsgemässen härtbaren Zusammensetzungen sind ein- und zweikomponentige Polyurethanzusammensetzungen zugänglich, die formal über primäre aromatische Diamine, insbesondere sterisch wenig oder nicht gehinderte primäre aromatische Diamine, welche als solche relativ hochschmelzende und oft schwerlösliche Feststoffe darstellen und deshalb in der Praxis kaum einsetzbar sind, aushärten. Insbesondere können die Polyurethanzusammensetzungen formal über das besonders hochschmelzende und schwerlösliche 1,4-Phenylendiamin ausgehärtet werden. Ausgehärtete Polyurethanzusammensetzungen mit Harnstoffbindungen, welche formal aus 1,4-Phenylendiamin und aromatischen Isocyanatgruppen aufgebaut sind, weisen eine besonders hohe Festigkeit und eine besonders gute Beständigkeit gegenüber Wärme und Feuchtigkeit auf.

Bevorzugte Anwendungen der beschriebenen härtbaren Zusammensetzung sind Klebstoffe, Dichtstoffe, Vergussmassen, Beschichtungen, Bodenbeläge, Anstriche, Lacke, Primer oder Schäume.

Speziell geeignet ist die beschriebene härtbare Zusammensetzung als einkomponentiger oder zweikomponentiger elastischer Klebstoff mit hohen Anforderungen an die Mechanik wie hohe Elastizitätsmodule bei einer hohen Dehnbarkeit, sowie für eine einkomponentige oder zweikomponentige Beschichtung, insbesondere ein Bodenbelag, mit hoher Rissüberbrückung.

Insbesondere geeignet ist die beschriebene härtbaren Zusammensetzung als Klebstoff im Fahrzeugbau, insbesondere für das Verkleben von Scheiben.

Ein weiterer Aspekt der vorliegenden Erfindung betrifft ein Verfahren zum Verkleben von einem Substrat **S1** mit einem Substrat **S2,** welches die Schritte umfasst:
i) Applikation einer vorgängig beschriebenen härtbaren Zusammensetzung auf ein Substrat **S1;**
ii) Kontaktieren der applizierten Zusammensetzung mit einem Substrat **S2** innerhalb der Offenzeit der Zusammensetzung;
   oder
i') Applikation einer vorgängig beschriebenen härtbaren Zusammensetzung auf ein Substrat **S1** und auf ein Substrat **S2;**
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

Ein weiterer Aspekt der vorliegenden Erfindung betrifft ein Verfahren zum Abdichten. Dieses umfasst den Schritt:
i") Applikation einer vorgängig beschriebenen härtbaren Zusammensetzung zwischen ein Substrat **S1** und ein Substrat **S2,** so dass die Zusammensetzung mit dem Substrat **S1** und dem Substrat **S2** in Kontakt steht;
   wobei das Substrat **S2** aus dem gleichen oder einem unterschiedlichen Material wie das Substrat **S1** besteht.
   Üblicherweise wird die Zusammensetzung in eine sogenannte Fuge eingepresst.

Ein weiterer Aspekt der vorliegenden Erfindung betrifft ein Verfahren zum Beschichten eines Substrates **S1.** Dieses umfasst den Schritt:
i"') Applikation einer vorgängig beschriebenen härtbaren Zusammensetzung auf ein Substrat **S1** innerhalb der Offenzeit der Zusammensetzung.

In diesen Verfahren sind geeignete Substrate **S1** und/oder **S2** beispielsweise anorganische Substrate wie Glas, Glaskeramik, Beton, Mörtel, Backstein, Ziegel, Gips und Natursteine wie Granit oder Marmor; Metalle oder Legierungen wie Aluminium, Stahl, Buntmetalle, verzinkte Metalle; organische Substrate wie Leder, Stoffe, Papier, Holz, mit Harz gebundene Holzwerkstoffe, Harz-Texil-Compositewerkstoffe, Kunststoffe wie Polyvinylchlorid (Hart- und Weich-PVC), Acrylonitril-Butadien-Styrol-Copolymere (ABS), SMC (Sheet Moulding Composites), Polycarbonat (PC), Polyamid (PA), Polyester, PMMA, Polyester, Epoxidharze, Polyurethane (PUR), Polyoxymethylen (POM), Polyolefine (PO), insbesondere mittels Plasma, Corona oder Flammen oberflächenbehandeltes Polyethylen (PE) oder Polypropylen (PP), Ethylen/Propylen-Copolymere (EPM) und Ethylen/Propylen-Dien-Terpolymere (EPDM); beschichtete Substrate wie pulverbeschichtete Metalle oder Legierungen; sowie Farben und Lacke, insbesondere Automobillacke.

Die Substrate können bei Bedarf vor dem Applizieren der Zusammensetzung vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.

Im Fall einer zweikomponentigen Zusammensetzung werden die beiden Komponenten **K1** und **K2** kurz vor der Applikation miteinander vermischt.

Der Auftrag der beschriebenen härtbaren Zusammensetzung zum Verkleben und/oder Abdichten von Substraten **S1** und/oder **S2** erfolgt typischerweise aus handelsüblichen Kartuschen, welche für kleinere Anwendungen bevorzugt manuell betrieben werden. Eine Applikation mittels Druckluft aus einer Kartusche oder aus einem Fass oder Hobbock mittels einer Förderpumpe oder eines Extruders, gegebenenfalls mittels eines Applikations-Roboters, ist ebenfalls möglich. Derartige Applikationsarten werden insbesondere in Anwendungen der industriellen Fertigung oder bei grossen Applikationen bevorzugt.

Die Applikation der härtbaren Zusammensetzung kann in einem breiten Temperaturspektrum erfolgen. Beispielsweise kann die Zusammensetzung bei Raumtemperatur appliziert werden, wie es für einen elastischen Klebstoff oder Dichtstoff typisch ist. Die Zusammensetzung kann aber auch bei tieferen wie auch bei höheren Temperaturen appliziert werden. Letzteres ist insbesondere dann vorteilhaft, wenn die Zusammensetzung anteilig hochviskose oder schmelzbare Komponenten enthält, wie sie in Schmelzklebstoffen, beispielsweise Warmschmelzklebstoffen (Warm-Melt) oder Heissschmelzklebstoffen (Hot-Melt) typischerweise vorhanden sind.

Aus den beschriebenen Verfahren zum Verkleben, Abdichten bzw. Beschichten - beziehungsweise aus der Verwendung einer der beschriebenen härtbaren Zusammensetzungen als Klebstoff, Dichtstoff, Vergussmasse, Beschichtung, Bodenbelag, Anstrich, Lack, Primer oder Schaum - entsteht ein Artikel.

Dieser Artikel ist insbesondere ein Bauwerk, insbesondere ein Bauwerk des Hoch- oder Tiefbaus, oder ein industrielles Gut oder ein Konsumgut, insbesondere ein Fenster, eine Haushaltmaschine, oder ein Transportmittel, insbesondere ein Fahrzeug zu Wasser oder zu Lande, bevorzugt ein Automobil, ein Bus, ein Lastkraftwagen, ein Zug oder ein Schiff, oder ein Anbauteil eines Transportmittels, oder ein Artikel der Möbel-, Textil- oder Verpackungsindustrie.

### Beispiele

### 1. Beschreibung der Messmethoden

**Infrarotspektren** wurden auf einem FT-IR Gerät 1600 von Perkin-Elmer als unverdünnte Filme auf einer horizontalen ATR-Messeinheit mit ZnSe-Kristall gemessen, wobei feste Substanzen zum Auftragen aufgeschmolzen wurden; die Absorptionsbanden sind in Wellenzahlen (cm⁻¹) angegeben (Messfenster: 4000-650 cm⁻¹); der Zusatz sh weist auf eine als Schulter erscheinende Bande hin, der Zusatz br auf eine breite Bande.

**¹H-NMR-Spektren** wurden auf einem Spektrometer des Typs Bruker DPX-300 bei 300.13 MHz gemessen; die chemischen Verschiebungen δ sind angegeben in ppm relativ zu Tetramethylsilan (TMS), Kopplungskonstanten *J* sind angegeben in Hz. Echte und Pseudo-Kopplungsmuster wurden nicht unterschieden.

Die **Viskosität** wurde auf einem thermostatisierten Kegel-Platten-Viskosimeter Physica UM (Kegeldurchmesser 20 mm, Kegelwinkel 1°, Kegelspitze-Platten-Abstand 0.05 mm, Scherrate 10 bis 1000 s⁻¹) gemessen, wie in DIN EN ISO 3219 beschrieben.

Der **Amingehalt,** das heisst der totale Gehalt an freien Aminogruppen und Aldiminogruppen in den hergestellten Dialdiminen, wurde titrimetrisch bestimmt (mit 0.1N HClO₄ in Eisessig, gegen Kristallviolett) und ist stets angegeben in mmol N/g.

### 2. Herstellung von Dialdiminen

### Beispiel 1: Dialdimin A-1

In einem Rundkolben wurden unter Stickstoffatmosphäre 5.00 g 2,4-Diaminotoluol (Amingehalt 16.33 mmol N/g; Schmelzpunkt 98-100 °C) in 24.26 g 2,2-Dimethyl-3-lauroyloxy-propanal suspendiert. Die Mischung wurde erwärmt und während 90 Minuten evakuiert (10 mbar, 80-100 °C), wobei eine homogene Flüssigkeit entstand. Ausbeute: 27.7 g eines rötlichbraunen, geruchsfreien Öls mit einem Amingehalt von 2.94 mmol N/g und einer Viskosität von 160 mPa·s bei 20 °C.
IR: 2954, 2922, 2852, 1736 (C=O), 1652 (C=N), 1600, 1572, 1492, 1466, 1419, 1392, 1374, 1366, 1349sh, 1302, 1246, 1156, 1110, 1074, 1022, 998, 934, 872, 822, 808sh, 766, 758, 720.
¹H-NMR (CDCl₃, 300 K): δ 7.73 und 7.66 (2×*s*, 2x1 H, CH=N), 7.09 (*dd, J* = 7.8 / 0.4, 1 H, Ar-H⁶), 6.71 (*dd*, *J* = 7.8 / 2.2, 1 H, Ar-H⁵), 6.40 (*d, J* = 2.2, 1 H, Ar-H³), 4.16 und 4.13 (2×*s*, 2x2 H, C(CH₃)₂-C*H*₂-O), 2.20 (s, 3 H, Ar-CH₃), 2.31 (2×*t, J* = 7.5, 4 H, OC(O)-C*H*₂-CH₂), 1.61 (*m*, 4 H, OC(O)-CH₂-C*H*₂), 1.23 (*m*, 32 H, CH₃-(C*H*₂)₈-CH₂-CH₂-CO), 1.22 (*s*, 12 H, C(C*H*₃)₂-CH₂-O), 0.88 (*t*, *J* = 6.7, 6 H, C*H*₃-(CH₂)₁₀-CO).

### Beispiel 2: Dialdimin A-2

In einem Rundkolben wurden unter Stickstoffatmosphäre 5.00 g 3,4-Diaminotoluol (Amingehalt 16.20 mmol N/g; Schmelzpunkt 87-89 °C) in 24.26 g 2,2-Dimethyl-3-lauroyloxy-propanal suspendiert. Die Mischung wurde erwärmt und während 90 Minuten evakuiert (10 mbar, 80 °C), wobei eine homogene Flüssigkeit entstand. Anschliessend wurde die Temperatur auf 100 °C erhöht und weitere 60 Minuten im Hochvakuum evakuiert. Ausbeute: 27.8 g eines dunkelbraunen, geruchsfreien Öls mit einem Amingehalt von 1.40 mmol N/g und einer Viskosität von 210 mPa·s bei 20 °C.
IR: 3370br, 2954, 2922, 2852, 1736 (C=O), 1652 (C=N), 1608, 1583, 1508, 1492, 1466, 1418, 1393, 1374, 1366sh, 1350sh, 1301, 1282sh, 1248, 1236, 1158, 1114, 1074, 1020, 1000, 932, 870, 834, 806, 778, 722.

### Beispiel 3: Dialdimin A-3

Unter gleichen Bedingungen wie in Beispiel 1 beschrieben wurden 5.00 g 1,3-Phenylendiamin (Amingehalt 18.43 mmol N/g; Schmelzpunkt 64-66 °C) mit 27.62 g 2,2-Dimethyl-3-lauroyloxy-propanal umgesetzt. Ausbeute: 30.7 g eines hellgelben, geruchsfreien Öls mit einem Amingehalt von 2.95 mmol N/g und einer Viskosität von 190 mPa·s bei 20 °C.
IR: 3060, 2955, 2922, 2852, 1736 (C=O), 1652 (C=N), 1592, 1466, 1418, 1392, 1374, 1366, 1349sh, 1302sh, 1282sh, 1248, 1236sh, 1156, 1112, 1076, 1022, 998, 934, 874, 816sh, 780, 722, 696.
¹H-NMR (CDCl₃, 300 K): δ 7.74 (s, 2 H, CH=N), 7.26 (*td*, *J* = 7.9 / 0.4, 1 H, Ar-H⁵), 6.81 (*dd*, *J* = 7.9/2.0, 2 H, Ar-H^{4,6}), 6.63 (*td, J* = 2.0/0.4, 1 H, Ar-H²), 4.14 (s, 4 H, C(CH₃)₂-C*H*₂-O), 2.31 (*t*, *J* = 7.5, 4 H, OC(O)-C*H*₂-CH₂), 1.61 (*m,* 4 H, OC(O)-CH₂-C*H*₂), 1.24 (m, 32 H, CH₃-(C*H*₂)₈-CH₂-CH₂-CO), 1.22 (s, 12 H, C(C*H*₃)₂-CH₂-O), 0.88 (*t*, *J* = 6.7, 6 H, C*H*₃-(CH₂)₁₀-CO).

### Beispiel 4: Dialdimin A-4

Unter gleichen Bedingungen wie in Beispiel 1 beschrieben wurden 5.00 g 1,4-Phenylendiamin (Amingehalt 18.40 mmol N/g; Schmelzpunkt 138-143 °C) mit 27.62 g 2,2-Dimethyl-3-lauroyloxy-propanal umgesetzt. Ausbeute: 30.7 g eines hellgelben, geruchsfreien Öls mit einem Amingehalt von 3.00 mmol N/g und einer Viskosität von 170 mPa·s bei 20 °C.
IR: 3031, 2954, 2922, 2852, 1736 (C=O), 1648 (C=N), 1604sh, 1573, 1496, 1466, 1418, 1392, 1374, 1366, 1347sh, 1298sh, 1282sh, 1249, 1231sh, 1206, 1156, 1110, 1072, 1022, 998, 936, 867, 846, 811, 770, 722.
¹H-NMR (CDCl₃, 300 K): δ 7.73 (s, 2 H, CH=N), 6.98 (s, 4 H, Ar-H), 4.14 (s, 4 H, C(CH₃)₂-C*H*₂-O), 2.31 (*t*, *J* = 7.5, 4 H, OC(O)-C*H*₂-CH₂), 1.61 (*m*, 4 H, OC(O)-CH₂-C*H*₂), 1.25 (*m*, 32 H, CH₃-(C*H*₂)₈-CH₂-CH₂-CO), 1.22 (s, 12 H, C(C*H*₃)₂-CH₂-O), 0.88 (*t, J* = 6.7, 6 H, CH₃-(C*H*₂)₁₀-CO).

### Beispiel 5: Dialdimin A-5

Unter gleichen Bedingungen wie in Beispiel 1 beschrieben wurden 10.00 g 4,4'-Diaminodiphenylmethan (Amingehalt 10.00 mmol N/g; Schmelzpunkt 89-91 °C) mit 29.87 g 2,2-Dimethyl-3-lauroyloxy-propanal umgesetzt. Ausbeute: 38.0 g eines bernsteinfarbenen, geruchsfreien Öls mit einem Amingehalt von 2.64 mmol N/g und einer Viskosität von 340 mPa·s bei 20 °C.
IR: 3022, 2954, 2922, 2851, 1735 (C=O), 1649 (C=N), 1606, 1572, 1504, 1466, 1416, 1392, 1373, 1366, 1295, 1248, 1208, 1158, 1110, 1074, 1016, 998,934,918,865,846,820,770,720.
¹H-NMR (CDCl₃, 300 K): δ 7.71 (s, 2 H, CH=N), 7.13 und 6.92 (2x*m*, 2x4 Ar-H), 4.13 (s, 4 H, C(CH₃)₂-C*H*₂-O), 3.95 (s, 2 H, Ar-CH₂-Ar), 2.30 (*t*, *J* = 7.5, 4 H, OC(O)-C*H*₂-CH₂), 1.60 (m, 4 H, OC(O)-CH₂-C*H*₂), 1.25 (*m*, 32 H, CH₃-(C*H*₂)₈-CH₂-CH₂-CO), 1.21 (s, 12 H, C(C*H*₃)₂-CH₂-O), 0.88 (*t*, *J* = 6.7, 6 H, C*H*₃-(CH₂)₁₀-CO).

### Beispiel 6: Dialdimin A-6

Unter gleichen Bedingungen wie in Beispiel 1 beschrieben wurden 10.00 g 4,4'-Diamino-3,3'-dichlor-diphenylmethan (MOCA; Amingehalt 7.43 mmol N/g; Schmelzpunkt 102-107 °C) mit 22.36 g 2,2-Dimethyl-3-lauroyloxy-propanal umgesetzt. Ausbeute: 31.1 g eines gelben, geruchsfreien Öls mit einem Amingehalt von 2.36 mmol N/g und einer Viskosität von 780 mPa·s bei 20 °C.
IR: 3468br, 3380br, 3027, 2953, 2922, 2852, 2716br, 1734 (C=O), 1658 (C=N), 1625, 1603sh, 1556, 1506, 1486, 1466, 1417, 1398, 1374, 1366, 1308, 1250, 1224, 1156, 1112, 1075, 1056, 1022, 1000, 932, 905, 896sh, 874, 830, 810, 774, 722, 694sh, 686.
¹H-NMR (CDCl₃, 300 K): δ 7.68 (*t*, *J* = 1.0, 2 H, CH=N), 7.16 (2x*d*, *J* = 1.7, 2 H, Ar-H²), 7.00 (*dd, J* = 8.0 / 1.7, 2 H, Ar-H⁶), 6.75 (*d, J* = 8.0, 2 H, Ar-H⁵), 4.15 (s, 4 H, C(CH₃)₂-C*H*₂-O), 3.87 (s, 2 H, Ar-CH₂-Ar), 2.31 (*t*, *J* = 7.5, 4 H, OC(O)-C*H*₂-CH₂), 1.61 (*m*, 4 H, OC(O)-CH₂-C*H*₂), 1.24 (*m*, 44 H, CH₃-(C*H*₂)₈-CH₂-CH₂-CO und C(C*H*₃)₂-CH₂-O), 0.88 (*t*, *J* = 6.7, 6 H, C*H*₃-(CH₂)₁₀-CO).

### Vergleichsbeispiel 7: Dialdimin A-7

In einem Rundkolben wurden unter Stickstoffatmosphäre 1.79 g 1,4-Phenylendiamin (1,4-PDA; Amingehalt 18.40 mmol N/g; Schmelzpunkt 138-143 °C) in 5.00 g 3-Acetoxy-2,2-dimethyl-propanal suspendiert. Dazu wurden 20 ml Dichlormethan gegeben und die Mischung während 10 Minuten bei Raumtemperatur gerührt, wobei sich das Amin vollständig löste und die anfänglich klare Lösung zunehmend eintrübte. Das Reaktionsgemisch wurde während 90 Minuten evakuiert (5·10⁻² mbar, 80-100 °C). Man erhielt 5.91 g eines hellbraunen, intensiv riechenden Festkörpers mit einem Amingehalt von 5.54 mmol N/g und einem Schmelzpunkt von 71-72 °C (Tottoli, unkorrigiert).

IR: 3027, 2968, 2943sh, 2870, 2846sh, 2715br, 1881br, 1736 (C=O), 1646 (C=N), 1600sh, 1570, 1494, 1470, 1435sh, 1394, 1372, 1228, 1104, 1038, 1008, 986, 928, 867, 842, 812, 793sh, 770, 728.

### Vergleichsbeispiel 8: Dialdimin A-8

Unter gleichen Bedingungen wie in Beispiel 1 beschrieben wurden 5.00 g trans-1,4-Diaminocyclohexan (Amingehalt 17.25 mmol N/g; Schmelzpunkt 68-72 °C) mit 25.76 g 2,2-Dimethyl-3-lauroyloxy-propanal umgesetzt. Ausbeute: 28.7 g eines bräunlichweissen, kristallinen und geruchsfreien Festkörpers mit einem Amingehalt von 2.92 mmol N/g und einem Schmelzpunkt von 47-50 °C (Tottoli, unkorrigiert).
IR: 2953sh, 2922, 2852, 2824sh, 1736 (C=O), 1664 (C=N), 1466, 1454sh, 1418, 1394, 1376, 1364sh, 1346, 1304, 1248, 1232, 1158, 1110, 1086, 1020, 1000, 942, 894, 887sh, 769, 722, 668.
¹H-NMR (CDCl₃, 300 K): δ 7.58 (s, 2 H, CH=N), 4.01 (s, 4 H, C(CH₃)₂-C*H*₂-O), 2.95 (*m*, 2 H, N-CH^{Cy}), 2.29 (*t*, *J* = 7.5, 4 H, OC(O)-C*H*₂-CH₂), 1.60 (*m*, 4 H, OC(O)-CH₂-C*H*₂), 1.26 (*m*, 32 H, CH₃-(C*H*₂)₈-CH₂-CH₂-CO), 1.08 (*s*, 12 H, C(C*H*₃)₂-CH₂-O), 0.88 (*t, J* = 6.7, 6 H, C*H*₃-(CH₂)₁₀-CO).

### Vergleichsbeispiel 9: Dialdimin A-9

In einem Rundkolben wurden unter Stickstoffatmosphäre 52.4 g (0.18 mol) destilliertes 2,2-Dimethyl-3-lauroyloxy-propanal vorgelegt. Unter kräftigem Rühren wurden aus einem beheizten Eintropftrichter 10.0 g (0.17 mol N) 1,6-Hexamethylendiamin (BASF; Amingehalt 17.0 mmol N/g) langsam zugegeben. Danach wurden die flüchtigen Bestandteile im Vakuum entfernt (10 mbar, 80 °C). Ausbeute: 57.7 g eines klaren, blassgelben und geruchsfreien Öls mit einem Amingehalt von 2.85 mmol N/g.

### Beispiel 10: Dialdimin A-10

Unter gleichen Bedingungen wie in Beispiel 1 beschrieben wurden 10.00 g 4,4'-Diaminodiphenylmethan (Amingehalt 10.00 mmol N/g; Schmelzpunkt 89-91 °C) mit 22.50 g 2,2-Dimethyl-3-heptanoyloxy-propanal umgesetzt. Ausbeute: 30.00 g eines bernsteinfarbenen, geruchsarmen Öls mit einem Amingehalt von 3.33 mmol N/g und einer Viskosität von 410 mPa·s bei 20 °C, welches bei 5 °C immer noch dünnflüssig war.
IR: 3024, 2956, 2926, 2858, 1734 (C=O), 1650 (C=N), 1606, 1572, 1504, 1468, 1458sh, 1436sh, 1416, 1392, 1374, 1366, 1316sh, 1294, 1234, 1208, 1160, 1102, 1054, 1025sh, 1016, 994, 934, 916, 865, 846, 820, 772, 726, 713sh.

### Vergleichsbeispiel 11: Dialdimin A-11

Als Vergleichsbeispiel wurde das Bespiel 10 von DE 31 33 769 A1 aus 4,4'-Diaminodiphenylmethan und 3-(2-Methylpropan-carbonyloxy-)2,2-dimethyl-propanal hergestellt. Es bildete sich ein bernsteinfarbenes, intensiv riechendes Öl mit einer Viskosität von 2040 mPa·s bei 20 °C, welches bei 5 °C honigartig war.

### 3. Herstellung von einkomponentigen Zusammensetzungen

### Beispiele 12 bis 18 und Vergleichsbeispiele 19 bis 21

In einem Polypropylenbecher mit Schraubverschluss wurde das Polymer ***P-1*,** dessen Herstellung nachfolgend beschrieben wird, mittels eines Zentrifugalmischers (SpeedMixer™ DAC 150, FlackTek Inc.; 1 min. bei 2500 U/min) mit den in der Tabelle 1 aufgeführten Zutaten in den angegebenen Gewichtsteilen zu einer homogenen Masse vermischt. Für das Vergleichsbeispiel 19 wurden das Polymer ***P-1*** und das Dialdimin ***A***-***8*** vor dem Vermischen auf 80 °C aufgeheizt, so dass das Dialdimin ***A-8*** flüssig vorlag.

Das Polymer ***P-1*** wurde wie folgt hergestellt:
4000 g Polyoxypropylen-Diol (Acclaim^{®} 4200 N, Bayer; OH-Zahl 28.5 mg KOH/g) und 520 g 4,4'-Methylendiphenyldiisocyanat (MDI, Desmodur^{®} 44 MC L, Bayer) wurden bei 80 °C zu einem NCO-terminierten Polyurethanpolymeren mit einem Gehalt an freien Isocyanatgruppen von 1.90 Gewichts-% umgesetzt.

In allen Dialdimin enthaltenden Zusammensetzungen beträgt das Verhältnis zwischen den Isocyanatgruppen und den Aldiminogruppen 1.0 / 0.7.

**Tabelle 1: Zusammensetzung der Beispiele 12 bis 18 und der Vergleichsbeispiele 19 bis 21. ^{a} 5 Gew.-% Salicylsäure in Dioctyladipat.**

| Beispiel | **12** | **13** | **14** | **15** | **16** | **17** | **18** | **19 (Vgl.)** | **20 (Vgl.)** | **21 (Vgl.)** |
|---|---|---|---|---|---|---|---|---|---|---|
| Polymer ***P-1*** | 50.0 | 50.0 | 50.0 | 50.0 | 50.0 | 50.0 | 50.0 | 50.0 | 50.0 | 50.0 |
| Dialdimin | ***A-1,*** 5.4 | ***A-2,*** 5.4 | ***A-3,*** 5.3 | ***A-4,*** 5.3 | ***A-5,*** 6.0 | ***A-6,*** 6.7 | ***A-10,*** 4.8 | ***A-8,*** 5.4 | - | ***A-11,*** 4.3 |
| Säure-Katalysator^{a} | 0.5 | 0.5 | 0.5 | 0.5 | 0.5 | 0.5 | 0.5 | 0.5 | 0.5 | 0.5 |

Die so erhaltenen Zusammensetzungen wurden auf Viskosität, Lagerstabilität, Hautbildungszeit, Aushärtungsgeschwindigkeit, Blasenbildung, mechanische Eigenschaften sowie Beständigkeit gegenüber thermischer Belastung geprüft.

Die **Lagerstabilität** wurde über die Veränderung (Zunahme) der Viskosität während der Lagerung in der Wärme bestimmt. Dazu wurde die Zusammensetzung in der verschlossenen Tube im Ofen bei 60 °C gelagert und die Viskosität bei 20 °C ein erstes Mal nach 6 Stunden und ein zweites Mal nach 7 Tagen Lagerdauer gemessen. Die Lagerstabilität ergibt sich aus der prozentualen Zunahme des zweiten Viskositätswerts gegenüber dem ersten.

Zur Messung der **Hautbildungszeit** (Zeit bis zur Klebefreiheit, "tackfree time") wurden einige Gramm der während 6 Stunden bei 60 °C gelagerten, raumtemperaturwarmen Zusammensetzung in einer Schichtdicke von ca. 2 mm auf Pappkarton aufgetragen und im Normklima (23±1 °C und 50±5% relative Luftfeuchtigkeit) die Zeit bestimmt, die es dauerte, bis beim leichten Antippen der Oberfläche der Zusammensetzung mittels einer Pipette aus LDPE erstmals keine Rückstände auf der Pipette mehr zurückblieben.

Als Mass für die **Aushärtungsgeschwindigkeit** wurde die Zeit bis zur Durchhärtung der Zusammensetzung herangezogen. Die Zeit bis zur **Durchhärtung** wurde bestimmt, indem die Zusammensetzung als Film in einer Schichtdicke von 5 mm in eine PTFE-Form gegossen, diese im Normklima gelagert und durch periodisches Anheben des Filmrands die Zeit in Tagen bestimmt wurde, die es dauerte, bis der Film sich erstmals rückstandsfrei von der Form ablösen liess.

Die **Blasenbildung** wurde qualitativ anhand der Menge Blasen, die während der Aushärtung der Zusammensetzung auftraten, beurteilt.

Als **mechanische Eigenschaften** wurden die **Zugfestigkeit** (Bruchkraft), die **Bruchdehnung** und das **E-Modul** der ausgehärteten Zusammensetzung gemessen. Dazu wurde die während 6 Stunden bei 60 °C gelagerte, raumtemperaturwarme Zusammensetzung in einer planen PTFE-Form zu einem Film von ca. 2 mm Dicke gegossen und dieser während 7 Tagen im Normklima ausgehärtet. Aus dem Film wurden Hanteln mit einer Länge von 75 mm, bei einer Steglänge von 30 mm und einer Stegbreite von 4 mm, ausgestanzt und gemäss DIN EN 53504 bei einer Zuggeschwindigkeit von 200 mm/min geprüft. Ein Teil der Hanteln wurde vor der Messung der mechanischen Eigenschaften während 7 Tagen im Ofen bei 100 °C und anschliessend 24 Stunden im Normklima gelagert, um die **Beständigkeit gegenüber thermischer Belastung** der ausgehärteten Zusammensetzung zu prüfen.

Die Ergebnisse der Prüfungen sind in der Tabelle 2 aufgeführt.

**Tabelle 2: Eigenschaften der Beispiele 12 bis 18 und der Vergleichsbeispiele 19 bis 21.**

| Beispiel | **12** | **13** | **14** | **15** | **16** | **17** | **18** | **19 (Vgl.)** | **20 (Vgl.)** | **21 (Vgl.)** |
|---|---|---|---|---|---|---|---|---|---|---|
| Viskosität nach 6h^{a} | 38 | 41 | 37 | 34 | 40 | 50 | 36 | 31 | 48 | 42 |
| Viskosität nach 7d^{a} | 45 | 73 | 40 | 37 | 44 | 66 | 40 | 33 | 52 | 49 |
| Δ_{Viskosität} (%)^{b} | 18 | 78 | 8 | 9 | 10 | 32 | 11 | 6 | 8 | 17 |
| Hautbildungszeit (min) | 230 | 300 | 270 | 45 | 85 | 900 | 145 | 15 | 360 | 175 |
| Durchhärtung (d) | 2.5 | 3 | 2 | 2 | 2 | 3.5 | 3 | 2.5 | 3 | 3 |
| Blasenbildung | keine | keine | keine | keine | keine | keine | keine | keine | viele | keine |
| Zugfestigkeit (MPa)^{c} | 3.1 | 1.8 | 6.7 | 6.3 | 3.0 | 3.0 | 3.5 | 6.0 | n.m. | 2.3 |
| | 3.7 | 1.7 | 5.7 | 10.3 | 3.4 | n.b. | n.b. | 4.0 | | n.b. |
| Bruchdehnung (%)^{c} | 880 | 560 | 960 | 950 | 870 | 970 | 990 | 650 | n.m. | 800 |
| | 870 | 530 | 1030 | 980 | 600 | n.b. | n.b. | 340 | | n.b. |
| E-Modul bei 0.5-5% Dehnung (MPa)^{c} | 4.2 | 3.4 | 3.9 | 13.0 | 8.6 | 1.8 | 6.8 | 12.7 | n.m. | 6.3 |
| | 3.9 | 3.3 | 4.2 | 11.5 | 8.8 | n.b. | n.b. | 9.9 | | n.b. |

| | | | | | | | | | | |
|---|---|---|---|---|---|---|---|---|---|---|
| ^{a} in Pa·s, nach Lagerung bei 60 °C, gemessen bei 20 °C. ^{b} Δ_{Viskosität} = [(Viskosität nach 7d / Viskosität nach 6h)- 1] × 100 %. ^{c}obere Zahl: Prüfung nach Aushärtung im Normklima; untere Zahl: Prüfung nach thermischer Belastung des ausgehärteten Materials (7d/100°C). n.b. = nicht bestimmt; n.m. = nicht messbar wegen zuvielen Blasen. | | | | | | | | | | |

Aus der Tabelle 2 ist ersichtlich, dass die erfindungsgemässen Zusammensetzungen der Beispiele 12 bis 18, welche von aromatischen Diaminen abgeleitete Dialdimine der Formel (I) enthalten, gegenüber den Zusammensetzungen des Vergleichsbeispiels 19, welches ein von einem cycloaliphatischen Diamin abgeleitetes Dialdimin enthält, eine deutlich längere Hautbildungszeit bei ähnlicher Aushärtungsgeschwindigkeit sowie eine bessere Beständigkeit gegenüber thermischer Belastung aufweisen. Die erfindungsgemässen Zusammensetzungen weisen eine genügende bis sehr gute Lagerstabilität auf, härten blasenfrei aus und verfügen über hervorragende mechanische Eigenschaften. Das Beispiel 15, welches ein Dialdimin von 1,4-Phenylendiamin enthält, zeichnet sich in seinem Eigenschaftsprofil ganz besonders aus.

### Beispiele 22 bis 26 und Vergleichsbeispiele 27 und 28

In einem Polypropylenbecher mit Schraubverschluss wurde das Polymer ***P-2*,** dessen Herstellung nachfolgend beschrieben wird, mittels eines Zentrifugalmischers (SpeedMixer™ DAC 150, FlackTek Inc.; 1 min. bei 2500 U/min) mit den in der Tabelle 3 aufgeführten Zutaten in den angegebenen Gewichtsteilen zu einer homogenen Masse vermischt.

Das Polymer ***P-2*** wurde wie folgt hergestellt:

1800 g entwässertes Polyoxypropylen-Diol (Acclaim^{®} 4200 N, Bayer; OH-Zahl 28.5 mg KOH/g), 500 g entwässertes Polyoxypropylen-Triol (Acclaim^{®} 6300, Bayer; OH-Zahl 28.0 mg KOH/g) und 200 g Toluylendiisocyanat (TDI; Desmodur^{®} T 80 P, Bayer) wurden bei 80 °C zu einem NCO-terminierten Polyurethanpolymeren mit einem Gehalt an freien Isocyanatgruppen von 1.89 Gewichts-% umgesetzt.

In allen Dialdimin enthaltenden Zusammensetzungen beträgt das Verhältnis zwischen den Isocyanatgruppen und den Aldiminogruppen 1.0 / 0.7.

**Tabelle 3: Zusammensetzung der Beispiele 22 bis 26 und der Vergleichsbeispiele 27 und 28.**

| Beispiel | **22** | **23** | **24** | **25** | **26** | **27 (Vgl.)** | **28 (Vgl.)** |
|---|---|---|---|---|---|---|---|
| Polymer ***P-2*** | 50.0 | 50.0 | 50.0 | 50.0 | 50.0 | 50.0 | 50.0 |
| Dialdimin | ***A-1,*** 5.3 | ***A-3,*** 5.3 | ***A-4,*** 5.2 | ***A-5,*** 6.0 | ***A-6,*** 6.7 | ***A-9,*** 5.5 | - |
| Säure-Katalysator^{a} | 0.5 | 0.5 | 0.5 | 0.5 | 0.5 | 0.5 | 0.5 |

| | | | | | | | |
|---|---|---|---|---|---|---|---|
| ^{a} 5 Gew.-% Salicylsäure in Dioctyladipat. | | | | | | | |

Die so erhaltenen Zusammensetzungen wurden wie für Beispiel 12 beschrieben auf Viskosität, Lagerstabilität, Hautbildungszeit, Blasenbildung, mechanische Eigenschaften sowie Beständigkeit gegenüber thermischer Belastung geprüft. Die Ergebnisse der Prüfungen sind in der Tabelle 4 aufgeführt.

**Tabelle 4: Eigenschaften der Beispiele 22 bis 26 und der Vergleichsbeispiele 27 und 28.**

| Beispiel | **22** | **23** | **24** | **25** | **26** | **27 (Vgl.)** | **28 (Vgl.)** |
|---|---|---|---|---|---|---|---|
| Viskosität nach 6h^{a} | 19.7 | 19.4 | 18.7 | 18.8 | 23.4 | 15.2 | 21.6 |
| Viskosität nach 7d^{a} | 23.0 | 21.0 | 20.6 | 20.1 | 26.7 | 17.2 | 23.7 |
| Δ_{Viskosität} (%)^{b} | 17 | 8 | 10 | 7 | 14 | 13 | 10 |
| Hautbildungszeit (min) | 450 | 360 | 80 | 210 | >720 | 20 | >720 |
| Blasenbildung | keine | keine | keine | keine | keine | keine | viele |
| Zugfestigkeit (MPa)^{c} | 4.7 | 3.4 | 7.0 | 7.5 | 4.4 | 6.7 | n.m. |
| | 4.8 | 5.0 | 7.6 | n.b. | n.b. | 5.0 | |
| Bruchdehnung (%)^{c} | 800 | 650 | 830 | 850 | 740 | 870 | n.m. |
| | 770 | 620 | 710 | n.b. | n.b. | 780 | |
| E-Modul bei 0.5-5% Dehnung (MPa)^{c} | 4.0 | 5.2 | 9.1 | 6.9 | 2.6 | 2.7 | n.m. |
| | 4.7 | 6.2 | 9.4 | n.b. | n.b. | 2.1 | |

| | | | | | | | |
|---|---|---|---|---|---|---|---|
| ^{a} in Pa·s, nach Lagerung bei 60 °C, gemessen bei 20 °C. ^{b} Δ_{Viskosität} = [(Viskosität nach 7d / Viskosität nach 6h)- 1] × 100 %. ^{c} obere Zahl: Prüfung nach Aushärtung im Normklima; untere Zahl: Prüfung nach thermischer Belastung des ausgehärteten Materials (7d/100 °C). n.b. = nicht bestimmt; n.m. = nicht messbar wegen zuvielen Blasen. | | | | | | | |

Aus der Tabelle 4 ist ersichtlich, dass die erfindungsgemässen Zusammensetzungen der Beispiele 22 bis 26, welche von aromatischen Diaminen abgeleitete Dialdimine der Formel (I) enthalten, gegenüber der Zusammensetzung des Vergleichsbeispiels 27, welches ein von einem aliphatischen Diamin abgeleitetes Dialdimin enthält, eine deutlich längere Hautbildungszeit, eine höhere mechanische Festigkeit sowie eine bessere Beständigkeit gegenüber thermischer Belastung aufweisen. Die erfindungsgemässen Zusammensetzungen weisen eine genügende bis sehr gute Lagerstabilität auf und härten blasenfrei aus. Das Beispiel 24, welches ein Dialdimin von 1,4-Phenylendiamin enthält, zeichnet sich in seinem Eigenschaftsprofil ganz besonders aus.

### Beispiele 29 und 30 und Vergleichsbeispiel 31

In einem Polypropylenbecher mit Schraubverschluss wurde das Polymer ***P-3*,** dessen Herstellung nachfolgend beschrieben wird, mittels eines Zentrifugalmischers (SpeedMixer™ DAC 150, FlackTek Inc.; 1 min. bei 2500 U/min) mit den in der Tabelle 5 aufgeführten Zutaten in den angegebenen Gewichtsteilen zu einer homogenen Masse vermischt.

Das Polymer ***P-3*** wurde wie folgt hergestellt:

590 g Polyoxypropylen-Diol (Acclaim^{®} 4200 N, Bayer; OH-Zahl 28.5 mg KOH/g), 1180 g Polyoxyethylenpolyoxypropylen-Triol (Caradol^{®} MD34-02, Shell; OH-Zahl 35.0 mg KOH/g) und 230 g Isophorondiisocyanat (IPDI; Vestanat^{®} IPDI, Degussa) wurden bei 80 °C in Anwesenheit von 0.01 g Dibutylzinn-dilaurat zu einem NCO-terminierten Polyurethanpolymeren mit einem Gehalt an freien Isocyanatgruppen von 2.10 Gewichts-% umgesetzt.

Das Verhältnis zwischen den Isocyanatgruppen und den Aldiminogruppen beträgt für alle Beispiele 1.0 / 0.7.

**Tabelle 5: Zusammensetzung der Beispiele 29 und 30 und des Vergleichsbeispiels 31.**

| Beispiel | **29** | **30** | **31 (Vergleich)** |
|---|---|---|---|
| Polymer ***P-3*** | 50.0 | 50.0 | 50.0 |
| Dialdimin | ***A-4,*** 5.8 | ***A-5,*** 6.6 | - |
| Säure-Katalysator^{a} | 0.5 | 0.5 | 0.5 |
| Zinn-Katalysator^{b} | - | - | 0.1 |

| | | | |
|---|---|---|---|
| ^{a} 5 Gew.-% Salicylsäure in Dioctyladipat. ^{b} 5 Gew.-% Dibutylzinndilaurat in Diisodecylphthalat. | | | |

Die so erhaltenen Zusammensetzungen wurden wie für Beispiel 12 beschrieben auf Viskosität, Lagerstabilität, Hautbildungszeit, Blasenbildung, mechanische Eigenschaften sowie Beständigkeit gegenüber thermischer Belastung geprüft. Die Ergebnisse der Prüfungen sind in der Tabelle 6 aufgeführt.

**Tabelle 6: Eigenschaften der Beispiele 29 und 30 und des Vergleichsbeispiels 31.**

| Beispiel | **29** | **30** | **31 (Vergleich)** |
|---|---|---|---|
| Viskosität nach 6h^{a} | 15.4 | 15.0 | 21.8 |
| Viskosität nach 7d^{a} | 15.5 | 15.8 | 22.4 |
| Δ_{Viskosität} (%)^{b} | 1 | 5 | 3 |
| Hautbildungszeit (min) | 480 | 480 | >720 |
| Blasenbildung | keine | keine | keine |
| Zugfestigkeit (MPa)^{c} | 2.4 | 2.5 | 1.1 |
| | 2.3 | 2.5 | n.m. |
| Bruchdehnung (%)^{c} | 400 | 460 | 310 |
| | 400 | 440 | n.m. |
| E-Modul bei 0.5-5% Dehnung (MPa)^{c} | 1.3 | 1.4 | 1.1 |
| | 1.1 | 1.3 | n.m. |

| | | | |
|---|---|---|---|
| ^{a} in Pa·s, Lagerung bei 60°C. ^{b} Δ_{Viskosität} = [(Viskosität nach 7d / Viskosität nach 6h) - 1] × 100 %. ^{c} obere Zahl: Prüfung nach Aushärtung im Normklima; untere Zahl: Prüfung nach thermischer Belastung des ausgehärteten Materials (7d/100 °C). n.m. = nicht messbar, da zersetzt. | | | |

Aus der Tabelle 6 ist ersichtlich, dass die erfindungsgemässen Zusammensetzungen der Beispiele 29 bis 30, welche von aromatischen Diaminen abgeleitete Dialdimine der Formel (I) enthalten, gegenüber der Zusammensetzung des Vergleichsbeispiels 31, welches kein Dialdimin enthält, eine kürzere Hautbildungszeit, eine höhere mechanische Festigkeit sowie eine bessere Beständigkeit gegenüber thermischer Belastung aufweisen. Die erfindungsgemässen Zusammensetzungen weisen eine sehr gute Lagerstabilität auf und härten blasenfrei aus.

### Beispiele 32 bis 36 und Vergleichsbeispiele 37 und 38: Elastische Klebstoffe (z.B. für die Scheibenverklebung)

Für jedes Beispiel wurden die jeweiligen Bestandteile gemäss Tabelle 7 in den angegebenen Gewichtsteilen in einem Vakuummischer unter Feuchtigkeitsausschluss zu einer homogenen Paste verarbeitet, diese unverzüglich in eine innenlackierte Aluminium-Kartusche abgefüllt und die Kartusche luftdicht verschlossen. Für das Vergleichsbeispiel 37 wurden das Polymer ***P-4*** und das Dialdimin ***A-8*** vor dem Vermischen auf 80 °C aufgeheizt, so dass das Dialdimin ***A-8*** flüssig vorlag.

Das Polymer ***P-4*** wurde wie folgt hergestellt:

1300 g Polyoxypropylen-Diol (Acclaim^{®} 4200 N, Bayer; OH-Zahl 28.5 mg KOH/g), 2600 g Polyoxypropylenpolyoxyethylen-Triol (Caradol^{®} MD34-02, Shell; OH-Zahl 35.0 mg KOH/g), 600 g 4,4'-Methylendiphenyldiisocyanat (MDI, Desmodur^{®} 44 MC L, Bayer) und 500 g Diisodecylphthalat (DIDP; Palatinol^{®} Z, BASF) wurden bei 80 °C zu einem NCO-terminierten Polyurethanpolymeren mit einem Gehalt an freien Isocyanat-Gruppen von 2.05 Gewichts-% umgesetzt.

Das Verhältnis zwischen den Isocyanatgruppen und den Aldiminogruppen beträgt für alle Beispiele 1.0 / 0.5.

**Tabelle 7: Zusammensetzung der elastischen Klebstoffe.**

| Beispiel | **32** | **33** | **34** | **35** | **36** | **37 (Vgl).** | **38 (Vgl).** |
|---|---|---|---|---|---|---|---|
| Polymer ***P-4*** | 50.00 | 50.00 | 50.00 | 50.00 | 50.00 | 50.00 | 50.00 |
| Dialdimin | ***A-1,*** 4.14 | ***A-2,*** 4.17 | ***A-3,*** 4.14 | ***A-4,*** 4.06 | ***A-5,*** 4.62 | ***A-8,*** 4.17 | ***A-9,*** 4.28 |
| Weichmacher^{a} | 9.86 | 9.83 | 9.86 | 9.94 | 9.38 | 9.83 | 9.72 |
| Kaolin^{b} | 17.50 | 17.50 | 17.50 | 17.50 | 17.50 | 17.50 | 17.50 |
| Russ^{b} | 17.50 | 17.50 | 17.50 | 17.50 | 17.50 | 17.50 | 17.50 |
| Trocknungsmittel^{c} | 0.20 | 0.20 | 0.20 | 0.20 | 0.20 | 0.20 | 0.20 |
| Epoxysilan^{d} | 0.30 | 0.30 | 0.30 | 0.30 | 0.30 | 0.30 | 0.30 |
| Säure-Katalysator^{e} | 0.50 | 0.50 | 0.50 | 0.50 | 0.50 | 0.50 | 0.50 |

| | | | | | | | |
|---|---|---|---|---|---|---|---|
| ^{a} Diisodecylphthalat (DIDP; Palatinol^{®} Z, BASF). ^{b} getrocknet bei 130 °C. ^{c} p-Toluolsulfonylisocyanat (Zusatzmittel TI, Bayer). ^{d} 3-Glycidoxypropyltriethoxysilan (Dynasylan^{®} GLYEO, Degussa). ^{e} 5 Gew.-% Salicylsäure in Dioctyladipat. | | | | | | | |

Die so erhaltenen elastischen Klebstoffe wurden auf Applikationseigenschaften, Hautbildungszeit, Aushärtungsgeschwindigkeit, mechanische Eigenschaften sowie Beständigkeit gegenüber thermischer und hydrolytischer Belastung geprüft.

Als Mass für die **Applikationseigenschaften** wurden die Standfestigkeit und der Fadenzug herangezogen. Zur Bestimmung der **Standfestigkeit** wurde der Klebstoff mittels Kartuschenpistole über eine Dreiecksdüse als waagrecht verlaufende Dreiecksraupe mit einem Basisdurchmesser von 8 mm und einer Höhe (Abstand der Dreiecksspitze von der Basis) von 20 mm auf ein senkrecht stehendes Stück Pappkarton aufgetragen. Nach 5 Minuten wurde gemessen, wie weit sich die Spitze abgesenkt, d.h. von der ursprünglichen Position in der Mitte der Dreiecksraupe wegbewegt, hatte. Als "sehr gut", beziehungsweise "s. gut", wurde bewertet, wenn sich die Spitze in vollständig oder annährend unveränderter Position befand, als "gut", wenn die Spitze sich zwischen Mitte und Basisende befand. Der **Fadenzug** wurde qualitativ bestimmt, indem etwas Klebstoff mittels Kartuschenpistole auf ein an der Wand befestigtes Stück Pappkarton aufgetragen wurde, die Kartuschenpistole beim Auftragsende durch rasches Zurückziehen vom aufgetragenen Klebstoff weggezogen und die Länge des dabei an der Abrissstelle zurückbleibenden Fadens gemessen wurde.

Als Mass für die **Aushärtungsgeschwindigkeit** wurde die Zeit bis zur Durchhärtung des Klebstoffs herangezogen. Die Zeit bis zur **Durchhärtung** wurde untersucht, indem der Klebstoff mittels Kartuschenpistole durch eine Rundspitze (Öffnung 10 mm) als waagrechter, frei hängender Konus mit einer Länge von ca. 50 mm und einer Dicke in der Mitte von 30 mm auf ein an der Wand befestigtes Stück Pappkarton aufgetragen wurde, während 7 Tagen im Normklima belassen, dann vertikal mittig aufgeschnitten und die Dicke der ausgehärteten Klebstoff-Schicht mit einem Massstab gemessen wurde.

Zur Bestimmung der mechanischen Eigenschaften nach der Aushärtung wurden die Shore A-Härte, die Zugfestigkeit, Bruchdehnung und das E-Modul gemessen. Die **Shore A-Härte** wurde bestimmt nach DIN 53505 an während 14 Tagen im Normklima ausgehärteten Prüfkörpern. Die **Zugfestigkeit, Bruchdehnung** und das **E-Modul** wurden bestimmt an während 7 Tagen im Normklima ausgehärteten Filmen mit einer Schichtdicke von 2 mm, auf die gleiche Weise wie bei Beispiel 12 beschrieben.

Zur Bestimmung der **Beständigkeit gegenüber hydrolytischer Belastung** wurden einige der für die Messung der mechanischen Eigenschaften vorbereiteten Hanteln während 7 Tagen unter "Kataplasma"-Bedingungen (70 °C/100% relative Luftfeuchtigkeit) und anschliessend 24 Stunden im Normklima gelagert, worauf die mechanischen Eigenschaften wie beschrieben geprüft wurden.

Die übrigen Eigenschaften wurden wie bei Beispiel 12 beschrieben geprüft.
Alle Klebstoffe härteten vollständig blasenfrei aus.
Die Ergebnisse der Prüfungen sind in der Tabelle 8 aufgeführt.

**Tabelle 8: Eigenschaften der elastischen Klebstoffe.**

| Beispiel | **32** | **33** | **34** | **35** | **36** | **37 (Vgl.)** | **38 (Vgl.)** |
|---|---|---|---|---|---|---|---|
| Standfestigkeit | s. gut | s. gut | gut | s. gut | s. gut | s. gut | gut |
| Fadenzug (cm) | 4 | 4 | 1.5 | 1.5 | 1.5 | 1.5 | 2.0 |
| Hautbildungszeit (min) | 245 | 150 | 155 | 105 | 120 | 30 | 35 |
| Durchhärtung (mm) | 11 | 8 | 12 | 12 | 12 | 9 | 8 |
| Shore A-Härte | 70 | 60 | 66 | 76 | 78 | 77 | 78 |
| Zugfestigkeit (MPa)^{a} | 8.7 | 8.2 | 8.8 | 8.7 | 9.1 | 8.6 | 8.8 |
| | 11.2 | 8.8 | 11.0 | 11.8 | 12.1 | 11.1 | 10.9 |
| | 10.4 | 8.9 | 10.7 | 11.0 | 11.2 | 10.2 | 10.3 |
| Bruchdehnung (%)^{a} | 460 | 520 | 440 | 320 | 380 | 310 | 320 |
| | 320 | 400 | 320 | 250 | 280 | 240 | 310 |
| | 310 | 470 | 330 | 280 | 310 | 260 | 290 |
| E-Modul bei 0.5-5% Dehnung (MPa)^{a} | 6.1 | 3.6 | 5.9 | 13.9 | 11.2 | 15.9 | 12.8 |
| | 8.3 | 3.6 | 7.7 | 15.9 | 14.0 | 18.1 | 13.7 |
| | 5.4 | 3.2 | 5.4 | 13.5 | 11.5 | 14.8 | 12.3 |

| | | | | | | | |
|---|---|---|---|---|---|---|---|
| ^{a} Obere Zahl: Prüfung nach Aushärtung im Normklima; mittlere Zahl: Prüfung nach thermischer Belastung des im Normklima ausgehärteten Materials (7d/100 °C); untere Zahl: Prüfung nach hydrolytischer Belastung ("Kataplasma") des im Normklima ausgehärteten Materials (7d/70 °C, 100%rF). | | | | | | | |

Aus der Tabelle 8 ist ersichtlich, dass die erfindungsgemässen Klebstoffe der Beispiele 32 bis 36, welche aromatische Dialdimine der Formel (I) enthalten, gegenüber den Klebstoffen der Vergleichsbeispiele 37 und 38, welche cycloaliphatische bzw. aliphatische Dialdimine enthalten, eine deutlich längere Hautbildungszeit und trotzdem eine hohe Aushärtungsgeschwindigkeit aufweisen. Die erfindungsgemässen Klebstoffe härten blasenfrei aus und verfügen über hervorragende mechanische Eigenschaften.

### Beispiele 39 bis 43 und Vergleichsbeispiel 44: Elastische Dichtstoffe (z.B. für die Fugenabdichtung)

Für jedes Beispiel wurden die jeweiligen Bestandteile gemäss Tabelle 9 in den angegebenen Gewichtsteilen in einem Vakuummischer unter Feuchtigkeitsausschluss zu einer homogenen Paste verarbeitet, diese unverzüglich in eine innenlackierte Aluminium-Kartusche abgefüllt und die Kartusche luftdicht verschlossen. Für das Vergleichsbeispiel 44 wurden das Polymer ***P-2*** und das Dialdimin ***A***-***8*** vor dem Vermischen auf 80 °C aufgeheizt, so dass das Dialdimin ***A-8*** flüssig vorlag.

Das Verdickungsmittel wurde wie folgt hergestellt:

In einem Vakuummischer wurden 3000 g Diisodecylphthalat (DIDP; Palatinol^{®} Z, BASF) und 480 g 4,4'-Methylendiphenyldiisocyanat (MDI, Desmodur^{®} 44 MC L, Bayer) vorgelegt und leicht aufgewärmt. Dann wurden unter starkem Rühren 270 g Monobutylamin langsam zugetropft. Die entstehende Paste wurde unter Vakuum und Kühlung eine Stunde weitergerührt.

Das Verhältnis zwischen den Isocyanatgruppen und den Aldiminogruppen beträgt für alle Beispiele 1.0 / 0.65.

**Tabelle 9: Zusammensetzung der elastischen Dichtstoffe.**

| Beispiel | **39** | **40** | **41** | **42** | **43** | **44 (Vgl.)** |
|---|---|---|---|---|---|---|
| Polymer ***P-2*** | 24.00 | 24.00 | 24.00 | 24.00 | 24.00 | 24.00 |
| Dialdimin | ***A-1,*** 2.77 | ***A-2,*** 2.79 | ***A-3,*** 2.77 | ***A-4,*** 2.71 | ***A-5,*** 3.09 | ***A***-***8***, 2.86 |
| Weichmacher^{a} | 0.83 | 0.81 | 0.83 | 0.89 | 0.51 | 1.74 |
| Kreide | 38.00 | 38.00 | 38.00 | 38.00 | 38.00 | 38.00 |
| Verdickungsmittel | 28.00 | 28.00 | 28.00 | 28.00 | 28.00 | 28.00 |
| Titandioxid | 4.50 | 4.50 | 4.50 | 4.50 | 4.50 | 4.50 |
| Trocknungsmittel^{b} | 0.15 | 0.15 | 0.15 | 0.15 | 0.15 | 0.15 |
| Epoxysilan^{c} | 0.25 | 0.25 | 0.25 | 0.25 | 0.25 | 0.25 |
| Säurekatalysator^{d} | 1.50 | 1.50 | 1.50 | 1.50 | 1.50 | 0.50 |

| | | | | | | |
|---|---|---|---|---|---|---|
| ^{a} Diisodecylphthalat (DIDP; Palatinol^{®} Z, BASF).^{b} Toluylendiisocyanat (TDI; Desmodur^{®} T 80 P, Bayer). ^{c} 3-Glycidoxypropyltriethoxysilan (Dynasylan^{®} GLYEO, Degussa). ^{d} Salicylsäure (5 Gew.-% in Dioctyladipat). | | | | | | |

Die so erhaltenen elastischen Dichtstoffe wurden auf Applikationseigenschaften, Hautbildungszeit und mechanische Eigenschaften nach der Aushärtung geprüft wie für Beispiel 32 beschrieben, wobei die Aushärtungszeit des Films für die mechanische Prüfung 14 Tage betrug und anstelle des E-Moduls die **Dehnspannung bei 100%** angegeben ist.

Weiterhin wurden die Dichtstoffe qualitativ auf **Klebrigkeit** geprüft. Dies erfolgte dadurch, dass man mit dem Daumen auf den aushärtenden Shore A-Prüfkörper, ein bzw. 3 Tage nach dessen Applikation, drückte und danach feststellte, wie lange der Prüfkörper beim Anheben der Hand am Daumen kleben blieb. Die Klebrigkeit wurde darauf als hoch (Prüfkörper bleibt mehr als 3 Sekunden lang kleben), mittel (Prüfkörper bleibt etwa 3 Sekunden lang kleben), gering (Prüfkörper bleibt 1 bis 2 Sekunden lang kleben) und keine (Prüfkörper bleibt weniger als 1 Sekunde lang kleben) bewertet.

Alle Dichtstoffe härteten vollständig blasenfrei aus.

Die Ergebnisse der Prüfungen sind in der Tabelle 10 aufgeführt.

**Tabelle 10: Eigenschaften der elastischen Dichtstoffe.**

| Beispiel | **39** | **40** | **41** | **42** | **43** | **44 (Vgl.)** |
|---|---|---|---|---|---|---|
| Standfestigkeit | s. gut | s. gut | s. gut | s. gut | s. gut | s. gut |
| Fadenzug (cm) | 1.0 | 0.5 | 0.8 | 0.8 | 1.5 | 1.5 |
| Hautbildungszeit (min) | 320 | 480 | 265 | 155 | 190 | 40 |
| Shore A-Härte | 39 | 29 | 40 | 52 | 38 | 51 |
| Zugfestigkeit (MPa) | 1.8 | 1.1 | 1.6 | 1.7 | 1.5 | 1.7 |
| Bruchdehnung (%) | 560 | 930 | 440 | 390 | 540 | 150 |
| Dehnspannung bei 100% (MPa) | 1.8 | 0.9 | 1.6 | 1.6 | 1.4 | 1.7 |
| Klebrigkeit nach 1 Tag | gering | hoch | keine | keine | gering | keine |
| Klebrigkeit nach 3 Tagen | keine | gering | keine | keine | keine | keine |

Aus der Tabelle 10 ist ersichtlich, dass die erfindungsgemässen Dichtstoffe der Beispiele 39 bis 43, welche aromatische Dialdimine der Formel (I) enthalten, gegenüber dem Dichtstoff des Vergleichsbeispiels 44, welcher ein cycloaliphatisches Dialdimin enthält, eine längere Hautbildungszeit und eine höhere Bruchdehnung aufweisen.

## Patentansprüche

1. Aromatisches, bei Raumtemperatur flüssiges, Dialdimin der Formel (I) 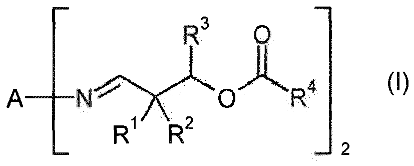 wobei
A für den Rest eines primären aromatischen Diamins **B** nach der Entfernung der zwei primären Aminogruppen steht;
das Diamin **B** ausgewählt ist aus der Gruppe bestehend aus 1,2-, 1,3-und 1,4-Phenylendiamin, 2,4- und 2,6-Toluylendiamin, 3,4-Toluylendiamin, 5-Isopropyl-2,4-toluylendiamin, 5-(tert.Butyl)-2,4-toluylendiamin, 4,6-Dialkyl-1,3-phenylendiamine mit Alkylgruppen, die insbesondere Methyl-, Ethyl-, Isopropyl- oder 1-Methylpropyl-Gruppen sind, 4,4'-, 2,4'-und 2,2'-Diaminodiphenylmethan, 3,3'-Dimethyl-4,4'-diaminodiphenylmethan, 3,3'-Dichlor-4,4'-diaminodiphenylmethan (MOCA), 3,3'-Di-tert.butyl-4,4'-diaminodiphenylmethan, 5,5'-Methylendianthranilsäure, Dimethyl-(5,5'-methylendianthranilat), 1,3-Propylen-bis-(4-aminobenzoat), 1,4-Butylen-bis-(4-aminobenzoat) und 1,2-Bis-(2-aminophenylthio)-ethan; R¹ und R²
entweder unabhängig voneinander jeweils für einen einwertigen Kohlenwasserstoffrest mit 1 bis 12 C-Atomen stehen;
oder
zusammen für einen zweiwertigen Kohlenwasserstoffrest mit 4 bis 20 C-Atomen stehen, der Teil eines, gegebenenfalls substituierten, carbocyclischen Rings mit 5 bis 8, bevorzugt 6, C-Atomen ist;
R³ für ein Wasserstoffatom oder für eine Alkylgruppe oder für eine Cycloalkylgruppe oder für eine Arylalkylgruppe steht, insbesondere mit 1 bis 12 C-Atomen;
R⁴ entweder für einen linearen oder verzweigten Alkylrest mit 6 bis 20 C-Atomen, gegebenenfalls mit cyclischen Anteilen und gegebenenfalls mit mindestens einem Heteroatom, steht;
oder R⁴ für einen einfach oder mehrfach ungesättigten, linearen oder verzweigten Kohlenwasserstoffrest mit 6 bis 20 C-Atomen steht; und wobei als Raumtemperatur eine Temperatur von 23°C bezeichnet wird.

2. Aromatisches, bei Raumtemperatur flüssiges, Dialdimin gemäss Anspruch 1, **dadurch gekennzeichnet, dass**
R⁴ entweder
für einen linearen oder verzweigten Alkylrest mit 11 bis 20 C-Atomen, gegebenenfalls mit cyclischen Anteilen und gegebenenfalls mit mindestens einem Heteroatom, steht;
oder
für einen einfach oder mehrfach ungesättigten, linearen oder verzweigten Kohlenwasserstoffrest mit 11 bis 20 C-Atomen steht.

3. Aromatisches, bei Raumtemperatur flüssiges, Dialdimin gemäss einem der vorhergehenden Ansprüche, **dadurch gekennzeichnet, dass** das Diamin **B** ausgewählt ist aus der Gruppe bestehend aus 1,2-, 1,3- und 1,4-Phenylendiamin, 2,4- und 2,6-Toluylendiamin, 3,4-Toluylendiamin, 4,4'-, 2,4'- und 2,2'-Diaminodiphenylmethan und 3,3'-Dichlor-4,4'-diaminodiphenylmethan.

4. Härtbare Zusammensetzung enthaltend mindestens ein aromatisches, bei Raumtemperatur flüssiges, Dialdimin gemäss einem der Ansprüche 1 bis 3 sowie mindestens ein Epoxidharz und/oder mindestens ein Isocyanat.

5. Härtbare Zusammensetzung gemäss Anspruch 4, **dadurch gekennzeichnet, dass** sie mindestens ein Polyisocyanat **P** und mindestens ein aromatisches, bei Raumtemperatur flüssiges, Dialdimin der Formel (I) gemäss einem der Ansprüche 1 bis 3 umfasst.

6. Härtbare Zusammensetzung gemäss Anspruch 5, **dadurch gekennzeichnet, dass** das Dialdimin der Formel (I) in einer derartigen Menge in der Zusammensetzung vorhanden ist, dass das Verhältnis zwischen der Anzahl der Aldiminogruppen und der Anzahl der Isocyanatgruppen 0.1 bis 1.1, insbesondere 0.15 bis 1.0, besonders bevorzugt 0.2 bis 0.9, beträgt.

7. Härtbare Zusammensetzung gemäss einem der Ansprüche 5 bis 6, **dadurch gekennzeichnet, dass** die Zusammensetzung zweikomponentig ist und aus einer Komponente **K1** und einer Komponente **K2** besteht, welche getrennt voneinander gelagert und erst kurz vor der Applikation miteinander vermischt werden.

8. Härtbare Zusammensetzung gemäss Anspruch 7, **dadurch gekennzeichnet, dass**
das Polyisocyanat **P** und das Dialdimin der Formel (I) Teil der Komponente **K1** sind,
während die Komponente **K2** gegenüber Isocyanatgruppen reaktive Verbindungen, insbesondere Wasser und/oder Polyole und/oder Polyamine und/oder Aminoalkohole und/oder Polythiole, enthält.

9. Härtbare Zusammensetzung gemäss Anspruch 7, **dadurch gekennzeichnet, dass**
das Polyisocyanat **P** Teil der Komponente **K1** ist,
während die Komponente **K2** das Dialdimin der Formel (I) sowie gegenüber Isocyanatgruppen reaktive Verbindungen, insbesondere Wasser und/oder Polyole und/oder Polyamine und/oder Aminoalkohole und/oder Polythiole, enthält.

10. Ausgehärtete Zusammensetzung, welche durch die Reaktion einer Zusammensetzung gemäss einem der Ansprüche 5 bis 6 mit Wasser, insbesondere in Form von Luftfeuchtigkeit, erhalten wird.

11. Ausgehärtete Zusammensetzung, welche durch die Vermischung der zwei Komponenten **K1** und **K2** einer Zusammensetzung gemäss einem der Ansprüche 7 bis 9, gegebenenfalls gefolgt von einer Reaktion mit Wasser, erhalten wird.

12. Verwendung einer härtbaren Zusammensetzung gemäss einem der Ansprüche 4 bis 9 als Klebstoff, Dichtstoff, Vergussmasse, Beschichtung, Bodenbelag, Anstrich, Lack, Primer oder Schaum.

13. Verwendung gemäss Anspruch 12 als Klebstoff im Fahrzeugbau, insbesondere für das Verkleben von Scheiben.

14. Verfahren zum Verkleben von einem Substrat **S1** mit einem Substrat **S2,** welches die Schritte umfasst
i) Applikation einer härtbaren Zusammensetzung gemäss einem der Ansprüche 4 bis 9 auf ein Substrat **S1;**
ii) Kontaktieren der applizierten Zusammensetzung mit einem Substrat **S2** innerhalb der Offenzeit der Zusammensetzung;
oder
i') Applikation einer härtbaren Zusammensetzung gemäss einem der Ansprüche 4 bis 9 auf ein Substrat **S1** und auf ein Substrat **S2;**
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

15. Verklebter Artikel, welcher durch ein Verfahren gemäss Anspruch 14 erhalten wird.

## Claims

1. An aromatic, room temperature liquid dialdimine of the formula (I) 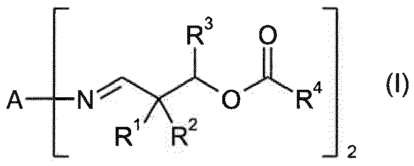 where
A is the radical of a primary aromatic diamine **B** after the removal of the two primary amino groups;
the diamine **B** is selected from the group consisting of 1,2-, 1,3- and 1,4-phenylenediamine, 2,4- and 2,6-tolylenediamine, 3,4-tolylenediamine, 5-isopropyl-2,4-tolylenediamine, 5-(tert-butyl)-2,4-tolylenediamine, 4,6-dialkyl-1,3-phenylenediamines with alkyl groups, which are especially methyl, ethyl, isopropyl or 1-methylpropyl groups, 4,4'-, 2,4'- and 2,2'-diaminodiphenylmethane, 3,3'-dimethyl-4,4'-diaminodiphenylmethane, 3,3'-dichloro-4,4'-diaminodiphenylmethane (MOCA), 3,3'-di-tert-butyl-4,4'-diaminodiphenylmethane, 5,5'-methylenedianthranilic acid, dimethyl 5,5'-methylenedianthranilate, 1,3-propylene bis(4-aminobenzoate), 1,4-butylene bis(4-aminobenzoate) and 1,2-bis(2-aminophenylthio)ethane;
R¹ and R²
are each independently either a monovalent hydrocarbon radical having 1 to 12 carbon atoms;
or
together are a divalent hydrocarbon radical which has 4 to 20 carbon atoms and is part of an optionally substituted carbocyclic ring having 5 to 8, preferably 6, carbon atoms;
R³ is a hydrogen atom or an alkyl group or a cycloalkyl group or an arylalkyl group, especially having 1 to 12 carbon atoms;
R⁴ is either a linear or branched alkyl radical having 6 to 20 carbon atoms, optionally having cyclic moieties and optionally having at least one heteroatom;
or R⁴ is a mono- or polyunsaturated, linear or branched hydrocarbon radical having 6 to 20 carbon atoms; and where room temperature refers to a temperature of 23°C.

2. An aromatic, room temperature liquid dialdimine as claimed in claim 1, **characterized in that**
R⁴ is either
a linear or branched alkyl radical having 11 to 20 carbon atoms, optionally having cyclic moieties and optionally having at least one heteroatom;
or
a mono- or polyunsaturated, linear or branched hydrocarbon radical having 11 to 20 carbon atoms.

3. An aromatic, room temperature liquid dialdimine as claimed in any of the preceding claims, **characterized in that** the diamine **B** is selected from the group consisting of 1,2-, 1,3- and 1,4-phenylenediamine, 2,4- and 2,6-tolylenediamine, 3,4-tolylenediamine, 4,4'-, 2,4'- and 2,2'-diaminodiphenylmethane and 3,3'-dichloro-4,4'-diaminodiphenylmethane.

4. A curable composition comprising at least one aromatic, room temperature liquid dialdimine as claimed in any of claims 1 to 3 and at least one epoxy resin and/or at least one isocyanate.

5. The curable composition as claimed in claim 4, **characterized in that** it comprises at least one polyisocyanate **P** and at least one aromatic, room temperature liquid dialdimine of the formula (I) as claimed in any of claims 1 to 3.

6. The curable composition as claimed in claim 5, **characterized in that** the dialdimine of the formula (I) is present in the composition in such an amount that the ratio between the number of aldimino groups and the number of isocyanate groups is 0.1 to 1.1, especially 0.15 to 1.0, more preferably 0.2 to 0.9.

7. The curable composition as claimed in any of claims 5 to 6, **characterized in that** the composition has two components and consists of a component **K1** and a component **K2** which are stored separately from one another and are mixed with one another only shortly before the application.

8. The curable composition as claimed in claim 7, **characterized in that** the polyisocyanate **P** and the dialdimine of the formula (I) are part of component **K1,**
while component **K2** comprises compounds reactive toward isocyanate groups, especially water and/or polyols and/or polyamines and/or amino alcohols and/or polythiols.

9. The curable composition as claimed in claim 7, **characterized in that** the polyisocyanate **P** is part of component **K1,**
while component **K2** comprises the dialdimine of the formula (I) and compounds reactive toward isocyanate groups, especially water and/or polyols and/or polyamines and/or amino alcohols and/or polythiols.

10. A cured composition which is obtained by the reaction of a composition as claimed in any of claims 5 to 6 with water, especially in the form of air humidity.

11. A cured composition which is obtained by mixing the two components **K1** and **K2** of a composition as claimed in any of claims 7 to 9, optionally followed by a reaction with water.

12. The use of a curable composition as claimed in any of claims 4 to 9 as an adhesive, sealant, potting composition, coating, floor covering, paint, lacquer, primer or foam.

13. The use as claimed in claim 12 as an adhesive in motor vehicle construction, especially for the adhesive bonding of window panes.

14. A process for adhesive bonding a substrate **S1** to a substrate **S2,** which comprises the steps of
i) applying a curable composition as claimed in any of claims 4 to 9 to a substrate **S1;**
ii) contacting the applied composition with a substrate **S2** within the open time of the composition; or
i') applying a curable composition as claimed in any of claims 4 to 9 to a substrate **S1** and to a substrate **S2;**
ii') contacting the applied compositions with one another within the open time of the composition;
where the substrate **S2** consists of the same material as, or a different material than, the substrate **S1.**

15. An adhesive bonded article which is obtained by a process as claimed in claim 14.

## Revendications

1. Dialdimine aromatique liquide à température ambiante répondant à la formule (I) 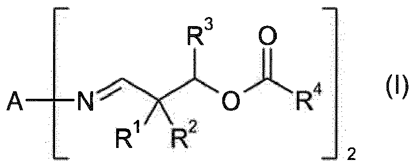 dans laquelle
A est le radical d'une diamine aromatique primaire **B** après élimination des deux groupes amino primaires ;
la diamine **B** est choisie dans le groupe constitué de la 1,2-, la 1,3- et la 1,4-phénylènediamine, la 2,4- et la 2,6-tolylènediamine, la 3,4-tolylènediamine, la 5-isopropyl-2,4-tolylènediamine, la 5-(tert-butyl)-2,4-tolylènediamine, les 4,6-dialkyl-1,3-phénylènediamines comportant des groupes alkyle qui sont en particulier des groupes méthyle, éthyle, isopropyle ou 1-méthylpropyle, le 4,4'-, 2,4'- et 2,2'-diaminodiphénylméthane, le 3,3'-diméthyl-4,4'-diaminodiphénylméthane, le 3,3'-dichloro-4,4'-diaminodiphénylméthane (MOCA), le 3,3'-di-tert-butyl-4,4'-diaminodiphénylméthane, l'acide 5,5'-méthylènedianthranilique, le diméthyle 5,5'-méthylènedianthranilate, le 1,3-propylène bis(4-aminobenzoate), le 1,4-butylène bis(4-aminobenzoate) et le 1,2-bis(2-aminophénylthio)éthane ;
R¹ et R²
représentent chacun, indépendamment l'un de l'autre, soit un radical hydrocarboné monovalent ayant de 1 à 12 atomes de carbone ;
soit
forment ensemble un radical hydrocarboné divalent qui a de 4 à 20 atomes de carbone et fait partie d'un cycle carbocyclique éventuellement substitué ayant de 5 à 8 et de préférence, 6 atomes de carbone ;
R³ est un atome d'hydrogène ou un groupe alkyle ou un groupe cycloalkyle ou un groupe arylalkyle, ayant en particulier de 1 à 12 atomes de carbone ;
R⁴ est soit un radical alkyle linéaire ou ramifié ayant de 6 à 20 atomes de carbone, ayant éventuellement des fractions cycliques et ayant éventuellement au moins un hétéroatome ;
soit R⁴ est un radical hydrocarboné linéaire ou ramifié mono- ou polyinsaturé ayant de 6 à 20 atomes de carbone ; et où la température ambiante correspond à une température de 23 °C.

2. Dialdimine aromatique liquide à température ambiante selon la revendication 1, **caractérisée en ce que**
R⁴ est soit
un radical alkyle linéaire ou ramifié ayant de 11 à 20 atomes de carbone, ayant éventuellement des fractions cycliques et ayant éventuellement au moins un hétéroatome ;
soit
un radical hydrocarboné linéaire ou ramifié mono- ou polyinsaturé ayant de 11 à 20 atomes de carbone.

3. Dialdimine aromatique liquide à température ambiante selon l'une quelconque des revendications précédentes, **caractérisée en ce que** la diamine **B** est choisie dans le groupe constitué de la 1,2-, la 1,3- et la 1,4-phénylènediamine, la 2,4- et la 2,6-tolylènediamine, la 3,4-tolylènediamine, le 4,4'-, le 2,4'- et le 2,2'-diamino-diphénylméthane et le 3,3'-dichloro-4,4'-diaminodiphénylméthane.

4. Composition durcissable comprenant au moins une dialdimine aromatique liquide à température ambiante selon l'une quelconque des revendications 1 à 3 et au moins une résine époxyde et/ou au moins un isocyanate.

5. Composition durcissable selon la revendication 4, **caractérisée en ce qu'**elle comprend au moins un polyisocyanate **P** et au moins une dialdimine aromatique liquide à température ambiante répondant à la formule (I) selon l'une quelconque des revendications 1 à 3.

6. Composition durcissable selon la revendication 5, **caractérisée en ce que** la dialdimine répondant à la formule (I) est présente dans la composition dans une quantité telle que le rapport entre le nombre de groupes aldimino et le nombre de groupes isocyanate est de 0,1 à 1,1, en particulier de 0,15 à 1,0 et de préférence encore de 0,2 à 0,9.

7. Composition durcissable selon l'une quelconque des revendications 5 et 6, **caractérisée en ce que** la composition a deux éléments et se compose d'un élément **K1** et d'un élément **K2** qui sont stockés séparément l'un de l'autre et mélangés l'un à l'autre peu de temps seulement avant l'application.

8. Composition durcissable selon la revendication 7, **caractérisée en ce que** le polyisocyanate **P** et la dialdimine répondant à la formule (I) font partie de l'élément **K1,**
alors que l'élément **K2** comprend des composés qui réagissent vis-à-vis des groupes isocyanate, en particulier de l'eau et/ou des polyols et/ou des polyamines et/ou des alcools aminés et/ou des polythiols.

9. Composition durcissable selon la revendication 7, **caractérisée en ce que** le polyisocyanate **P** fait partie de l'élément **K1,**
alors que l'élément **K2** comprend la dialdimine répondant à la formule (I) et des composés qui réagissent vis-à-vis des groupes isocyanate, en particulier de l'eau et/ou des polyols et/ou des polyamines et/ou des alcools aminés et/ou des polythiols.

10. Composition durcie qui s'obtient par réaction d'une composition selon l'une quelconque des revendications 5 à 6 avec de l'eau, en particulier sous forme d'humidité de l'air.

11. Composition durcie qui s'obtient par mélange des deux éléments **K1** et **K2** d'une composition selon l'une quelconque des revendications 7 à 9, éventuellement suivi d'une réaction avec de l'eau.

12. Utilisation d'une composition durcissable selon l'une quelconque des revendications 4 à 9 comme adhésif, produit d'étanchéité, composition d'enrobage, revêtement, revêtement de sol, peinture, vernis, apprêt ou mousse.

13. Utilisation selon la revendication 12 comme adhésif dans la construction automobile, en particulier pour le collage de vitres.

14. Procédé de collage d'un substrat **S1** sur un substrat **S2,** qui comprend les étapes suivantes
i) l'application d'une composition durcissable selon l'une quelconque des revendications 4 à 9 sur un substrat S1 ;
ii) la mise en contact de la composition appliquée avec un substrat **S2** dans le temps d'ouverture de la composition ; ou
i') l'application d'une composition durcissable selon l'une quelconque des revendications 4 à 9 sur un substrat **S1** et sur un substrat **S2 ;**
ii') la mise en contact des compositions appliquées l'une avec l'autre dans le temps d'ouverture de la composition ;
le substrat **S2** étant constitué d'un matériau identique à celui du substrat **S1** ou différent de celui-ci.

15. Article collé qui s'obtient par un procédé selon la revendication 14.
